# EUROPEAN PATENT SPECIFICATION

(11) **EP 1 616 000 B1**
(45) Date of publication and mention of the grant of the patent: **15.06.2011**
(21) Application number: 04727532.6
(22) Date of filing: 15.04.2004
(51) Int. Cl.: C12N 5/07, A61K 48/00, C07K 14/755

(54) **EXPRESSION OF PROTEINS IN CORD BLOOD-DERIVED ENDOTHELIAL CELLS**
EXPRESSION VON PROTEINEN IN AUS NABELSCHNURBLUT GEWONNENEN ENDOTHELZELLEN
EXPRESSION DE PROTEINES DANS DES CELLULES ENDOTHELIALES DERIVEES DE SANG OMBILICAL

(30) Priority: 15.04.2003 EP 03008076
(43) Date of publication of application: 18.01.2006
(73) Proprietor: DRK-Blutspendedienst Baden-Württemberg-Hessen gGmbH, 68167 Mannheim (DE)
(72) Inventor: HERDER, Christian, 40217 Düsseldorf (DE); TONN, Torsten, 60599 Frankfurt am Main (DE); GREZ, Manuel, 69121 Heidelberg (DE); SEIFRIED, Erhard, 61462 Königstein (DE)
(74) Representative: Kalhammer, Georg
(86) International application number: PCT/EP2004/003998
(87) International publication number: WO 2004/092355

(56) References cited:
- WO-A-02/08389
- WO-A-03/013570
- WO-A-03/070083
- ROSENBERG JONATHAN B ET AL: "Genetic induction of a releasable pool of factor VIII in human endothelial cells." ARTERIOSCLEROSIS THROMBOSIS AND VASCULAR BIOLOGY, vol. 20, no. 12, December 2000 (2000-12), pages 2689-2695, XP002254111 ISSN: 1079-5642 cited in the application
- CHUAH M K L ET AL: "DEVELOPMENT AND ANALYSIS OF RETROVIRAL VECTORS EXPRESSING HUMAN FACTOR VIII AS A POTENTIAL GENE THERAPY FOR HEMOPHILIA A" HUMAN GENE THERAPY, XX, XX, vol. 6, no. 11, 1 November 1995 (1995-11-01), pages 1363-1377, XP000570044 ISSN: 1043-0342 cited in the application
- FERRARI N ET AL: "Bone marrow-derived, endothelial progenitor-like cells as angiogenesis-selective gene-targeting vectors." GENE THERAPY, vol. 10, no. 8, 1 April 2003 (2003-04-01), pages 647-656, XP002254112 ISSN: 0969-7128
- TONN T ET AL: "Generation and characterization of human hematopoietic cell lines expressing factor VIII." JOURNAL OF HEMATOTHERAPY & STEM CELL RESEARCH. UNITED STATES AUG 2002, vol. 11, no. 4, August 2002 (2002-08), pages 695-704, XP009017042 ISSN: 1525-8165 cited in the application
- GEHLING URSULA M ET AL: "In vitro differentiation of endothelial cells from AC133-positive progenitor cells." BLOOD, vol. 95, no. 10, 15 May 2000 (2000-05-15), pages 3106-3112, XP002254113 ISSN: 0006-4971 cited in the application

## Description

The invention relates to a method for the production of a protein, e.g., a blood coagulation factor. The method comprises culturing endothelial cells derived from human cord blood and isolating the desired protein from the cell culture medium. The application further describes a population of human endothelial cells transduced with a vector encoding a protein, e.g., a blood coagulation factor. The cells can be used in the method for the production of a protein or in a gene therapy protocol.

Hemophilia A is caused by insufficient levels or even the complete absence of functional coagulation factor VIII (FVIII) in the circulation. It is an X chromosome-linked bleeding disorder that affects 1 in 5,000 to 10,000 males. FVIII is an essential component of the intrinsic pathway of the blood coagulation cascade, where it serves as a cofactor for activated factor IX within a membrane-bound complex (Xase complex) that activates factor X, which in its turn participates in conversion of the zymogen prothrombin into the enzyme thrombin. FVIII is synthesized as a 2332 amino acid residue molecule (∼300 kDa) consisting of three homologous A domains, two homologous C domains, and a unique B domain arranged in the order A1-A2-B-A3-C1-C2. FVIII is processed by multiple intracellular cleavages within the B domain, and at the B-A3 junction, to a heterodimer consisting of Me²⁺-linked light and heavy chains. The heavy chain is comprised of the A1 (1-336), A2 (373-740) and B domains (741-1648) and the light chain includes the A3 (1649-2019), C1 (2020-2172) and C2 (2173-2332) domains. In the circulation, FVIII is lightly bound to von Willebrand factor (VWF), a protein required for maintaining the normal FVIII level in plasma. VWF prevents premature formation of the Xase complex and protects FVIII from inactivation by activated protein C, activated factor IX (FIXa) and activated factor X (FXa). VWF deficiency in both humans and animals has been shown to lead to a secondary deficiency of FVIII, thus suggesting that VWF also prevents FVIII from accelerated clearance.

The main symptoms of hemophilia A are bleedings into joints, muscles and internal organs that can occur spontaneously and be life-threatening (Mannucci and Tuddenham, 2001; see infra for bibliographic data of the references). Based on the residual activity of FVIII in plasma, hemophilia A is categorized as severe (<1% of normal activity), moderate (1-5%) and mild (5-30%).

Hemophilia B occurs in about 1 of 25,000 males. It is characterized by the deficiency of the serine protease factor IX (Christmas factor). This 415 amino acid polypeptide is synthesized in the liver as a 56 kDa glycoprotein. In order to attain its proper function a posttranslational carboxylation step is required which only occurs in the presence of vitamin K.

Hemophilia A and B patients are currently treated by intravenous infusions of plasma-derived or recombinant FVIII and FIX, respectively (Mannucci and Giangrande, 2000). Although this substitution therapy has become relatively safe and efficacious during the last decade, there are several drawbacks such as the inconvenience of lifelong infusions and the potential transmission of infectious diseases by FVIII concentrates (Hoots, 2001; Teitel, 2000; White II *et al.,* 2000; VandenDriessche *et al.,* 2001). Due to a relatively short half-life of FVIII in the circulation (12-14 h), prophylactic treatment of hemophilia A requires repeated - up to three times a week - infusions of FVI preparations.

The use of non-human cell lines for the production of recombinant factor VIII encountered certain disadvantages. The major limitation in production of recombinant FVIII is low yield from FVIII expressing cells, which is two orders of magnitude lower than that for other proteins.

Several *in vivo* studies for the gene therapy of hemophilia A with viral and non-viral vectors were performed (reviewed in White II, 2001; Chuah *et al.,* 2001; Greengard and Jolly 1999, VandenDriessche *et al.,* 2001; Kaufman, 1999). However, there remain concerns over the safety of these approaches. Potential side effects include adverse immunological reactions, vector mediated cytotoxicity (Yang *et al.,* 1996; Lozier *et al.,* 1999) and germ-line transmission.

Under physiological conditions FVIII synthesis mainly occurs in hepatocytes and liver sinusoidal endothelial cells (Wion *et al.,* 1985; Zelechowska *et al.,* 1985; Do *et al.,* 1999; Hollestelle *et* a/., 2001). These and other cell types such as skin (Hoeben *et al.,* 1990; Fakharzadeh *et al.,* 2000), endothelial cells (Dwarki *et al.,* 1995; Chuah *et al.,* 1995; Rosenberg *et al.,* 2000), hepatocytes (Andrews *et al.,* 1999), bone marrow stromal cells (Chuah *et al.,* 1998) and hematopoietic cells (Hoeben *et al*., 1992; Evans & Morgan, 1998; Tonn *et al.,* 2002) may be useful in gene therapeutic approaches. Indeed, the implantation of *ex vivo* modified fibroblasts that secreted FVIII was shown to be well tolerated and led to detectable FVIII plasma levels in patients with severe hemophilia A (Roth *et al.,* 2001).

US patent application 2002/0042130 A1 and Lin et al. (2002) describe studies on the use of blood outgrowth endothelial cells (BOECs) for gene therapy for hemophilia A. Gehling et al. (2000) report the in vitro differentiation of endothelial cells from AC133-positive progenitor cells. Gehling et al. do not disclose, however, the transduction of cells with DNA encoding factor VIII. EP 1 136 553 A1 and WO 01/70968 A2 relate to the production of recombinant blood clotting factors in immortalized human cell lines.

Rosenberg et al. (2000) "Arteriosclerosis, thrombosis and vascular biology" 20, 2689-2695 describes the transduction of the B-domain deleted VIII gene in primary human umbilical vein endothelial cells (HUVECs). These cells coat the inner wall of the umbilical vein and thus represent fully differentiated endothelial cells which show very limited proliferative potential. Rosenberg et al. does not disclose the use of cells derived from cord blood, i.e. placental residual blood. The cells described by Rosenberg et al. differ from endothelial cells from cord blood in their phenotype and proliferative potential.

Ferrari et al. (2003), Gene Therapy 10, 647-656 describes studies on bone marrow-derived, endothelial progenitor-like cells as angiogenesis-selective gene-targeting vectors. The cells described in Ferrari et al. express β-gal, GFP or thymidine kinase.

WO 02/08389 A2 refers to therapeutic angiogenesis by bone marrow-derived cell transplantation in myocardial ischemic tissue and skeletal muscle ischemic tissue.

WO 03/013570 A1 relates to platelet-derived growth factor protection of cardiac myocardium and aims at providing methods of treating and preventing loss of tissue vascularisation that can occur upon aging. The cells according to WO 03/013570 A1 express PDGF.

Müller et al. (2002) investigates the expression of the endothelial markers PECAM-1, vWf, and CD34 in vitro and in vivo.

It is an object of the present invention to provide cells that are suitable for producing sufficient amounts of a therapeutically effective protein. It is a further object of the invention to provide cells that are suitable for producing sufficient amounts of functional factor VIII protein and can be used in gene therapy.

It has surprisingly been found that endothelial precursor cells derived from human cord blood can be differentiated into mature endothelial cells that secrete high levels of factor VIII upon viral transduction with cDNA encoding factor VIII. The invention therefore relates to a method for the preparation of human endothelial cells expressing a blood coagulation factor, comprising
a) obtaining endothelial precursor cells from human cord blood;
b) contacting in vitro said human cord blood-derived endothelial precursor cells with at least one growth factor, wherein the at least one growth factor promotes the differentiation of endothelial precursor cells into mature endothelial cells; and
c) transducing the mature endothelial cells with DNA encoding the blood coagulation factor, or transducing, prior to step b), the endothelial precursor cells with DNA encoding the blood coagulation factor.

The present invention further relates to a method for the production of a blood coagulation factor, comprising
a) obtaining a population of human endothelial cells by a method according to any one of claims 1-5; and optionally generating an immortalized cell line from said population of human endothelial cells;
b) culturing said population of human endothelial cells or said cell line under suitable conditions; and
c) isolating the blood coagulation factor from the cell culture medium.

The cells that are used in the method according to the invention are derived from cord blood.

The term "growth factor" refers to a substance, e.g., a protein, that is capable of inducing proliferation and/or differentiation of mammalian cells.

A "population of cells" designates a composition comprising at least two cells. The population generally comprises at least 100 cells, preferably at least 1000 cells. The cells may be homogeneous in respect to expression of a given marker protein, they may also be heterogeneous with respect to expression of another marker protein.

As used herein, the term "endothelial cell" or "mature endothelial cell" designates a cell that expresses the markers VE-cadherin (CD144), CD146, CD31 and LDL-receptor. "Endothelial cells" or "mature endothelial cells" may still have significant proliferative potential greater than that of fully differentiated endothelial cells.

A "differentiated endothelial cell" or "fully differentiated endothelial cell" refers to an endothelial cell that has undergone terminal differentiation. Differentiated endothelial cells thus have very limited proliferative potential.

An "endothelial precursor cell" (EPC) is a cell that can be differentiated into a mature endothelial cell. An EPC usually expresses CD34, AC133 and/or fibroblast growth factor 1 receptor (FGF1-R) and is characterized by its proliferative potential.

In a "population of endothelial cells" at least 10% of the cells are endothelial cells, preferably at least 25%, more preferably at least 50%, most preferably at least 75%.

As used herein, the term "CD34-positive cells" denotes cells that express the surface marker protein CD34. Expression of CD34 can be determined by immunofluorescence analysis or FACS analysis using an antibody directed against CD34.

A "blood coagulation factor" is a substance, e.g. a protein, which has the coagulant activity of a component of the intrinsic pathway of the blood coagulation cascade. Components of the blood coagulation cascade include, but are not limited to, factor V, factor VII, factor VIII, factor IX, VWF, and the like. "Factor VIII" denotes a substance, e.g. a protein, that has the ability, when administered to patients with Hemophilia A, to correct the clotting defect. As non-limiting examples, this definition includes full length recombinant factor VIII and B domain deleted factor VIII. "Factor IX" denotes a substance, e.g. a protein, that has the ability, when administered to patients with Hemophilia B, to correct the clotting defect.

The term "transducing" refers to a process wherein DNA is transferred into a cell. Viral or non-viral vectors may be used in this process. The cells are preferably human cells. The DNA may or may not integrate into the genome of the cell. For transduction, vectors may be used which contain a sequence encoding a polypeptide to be expressed. A promoter is usually operably linked to the coding sequence, i.e. the promoter is located within the vector in a manner that it can stimulate transcription of the DNA sequence encoding the protein, e.g. the blood coagulation factor.

The term "recombinant DNA" denotes a DNA molecule that has been prepared by joining DNA fragments from different sources. Such recombinant DNA molecules include cDNA and genomic clones. Recombinant DNA has been made by human intervention. Recombinant DNA molecules in accordance with the present invention are in a form suitable for use within genetically engineered protein production systems. The DNA may include naturally occurring 5'- and 3'-untranslated regions such as promoters and terminators.

The human endothelial precursor cells are derived from cord blood. The endothelial precursor cells may express at least one surface marker selected from the group consisting of CD34, AC133, CD146 and FGF1-R. In a particular embodiment the endothelial precursor cells express at least two, alternatively at least three, alternatively all of the said four cell surface marker proteins. Usually at least 10% of the population of endothelial precursor cells are positive for one of the above mentioned cell surface markers, preferably at least 25%, more preferably at least 50%, even more preferably at least 75%, most preferably at least 80%. The endothelial precursor cells may be obtained by enriching cells that are positive for one of the above mentioned cell surface markers. In a first step, mononuclear cells may be isolated from cord blood by Ficoll density separation. After washing of the mononuclear cells CD34-positive cells or AC133-postive cells or CD146-positive cells or FGF1-R-positive cells can be enriched using magnetic activated cell sorting. After a first step of enrichment, a second step of enrichment may be performed using another cell surface marker protein as target.

Bone marrow cells may be obtained by aspiration, and suitable endothelial precursor cells may be selected and amplified by suitable methods as described herein. Alternatively, human endothelial precursor cells obtained from cord blood and bone marrow may be mixed-. -

When CD34-positive cells are to be contacted with at least one growth factor, these cells may be obtained by enriching CD34-positive cells from human cord blood. Suitable enrichment techniques are known to those skilled in the art. In a first step, mononuclear cells may be isolated from blood by Ficoll density separation. After washing of the mononuclear cells, CD34-positive cells can be enriched using magnetic activated cell sorting. Using this method, the fraction of CD34-positive cells can be enriched from less than 2% in the mononuclear cell fraction to more than 80% in the enriched fraction. Isolation of CD34-positive cells by fluorescence-activated cell sorting (FACS) may also be contemplated.

In the CD34-positive cells that are contacted with at least one growth factor usually at least 10% of the cells are CD34-positive, preferably at least 25%, more preferably at least 50%, even more preferably at least 75%, most preferably at least 80%. In a preferred embodiment, the purity of CD34-positive cells is in the range of 80% to 95%. Populations of cells with purities of CD34-positive cells in this range can be obtained by enriching the cells as described above.

The cells may be cultured in an appropriate medium in tissue-culture plates or flasks prior to contacting them with the growth factor(s) for differentiation into endothelial cells.

According to the method of the invention, the cord blood-derived endothelial precursor cells are contacted in vitro with at least one growth factor, wherein the at least one growth factor promotes the differentiation of endothelial precursor cells into mature endothelial cells. Whether a growth factor promotes the differentiation of endothelial precursor cells into mature endothelial cells can be determined by contacting CD34-positive cells derived from cord blood with a growth factor and determining whether one or more of the endothelial markers CD144, CD146, CD31 and LDL-receptor are expressed.

In one embodiment, the cells are contacted with vascular endothelial growth factor (VEGF). In another embodiment, VEGF may be combined with another growth factor, e.g., basic fibroblast growth factor (bFGF), stem cell factor (SCF or stem cell growth factor-β (SCGF-β). The endothelial precursor cells may be contacted with one of the following combinations of growth factors: VEGF + bFGF; VEGF + SCF; VEGF + SCGF-β; VEGF + bFGF + SCF; VEGF + bFGF + SCGF-β; VEGF + SCF + SCGF-β. Most preferably, the endothelial precursor cells are contacted with the four growth factors VEGF, bFGF, SCF and SCGF-β. Usually, the growth factors are added to the cell culture medium. Preferably, the growth factors are human growth factors. The growth factors may be produced by recombinant expression.

The amino acid sequences and cDNA sequences of human VEGF, human basic FGF, human SCF and human SCGF- β are known (VEGF: Conn et al., Proc Natl Acad Sci, USA 1990 April 87(7):2628-32; Tischer E. et al., J Biol Chem 1991 June 25, 266(18):11947-54; bFGF: Kurokawa T. et al., FEBS Lett. 1987 March 9, 213(1):189-94; SCF: Martin F.H. et al., Cell 1990 October 5, 63(1):203-11; SCGF-β: Hiraoka A. et al., Proc Natl Acad Sci, USA 1997 July 8, 94(14):7577-82; Bannwarth S. et al., J Biol Chem 1998 January 23, 273(4):1911-6.

VEGF can be administered to the cell culture medium of the EPCs at a concentration of 1 to 1000 ng/ml, preferably 10 to 200 ng/ml, more preferably 25 to 100 ng/ml, most preferably about 50 ng/ml. Basic FGF is usually added to the medium at a concentration of 1 to 500 ng/ml, preferably 5 to 100 ng/ml, more preferably 10 to 50 ng/ml, most preferably about 20 ng/ml. SCF may be added at a concentration of 1 to 1000 ng/ml, preferably 10 to 200 ng/ml, more preferably 25 to 100 ng/ml, most preferably about 50 ng/ml. SCGF-β can be added to the medium at a concentration of 1 to 500 ng/ml, preferably 5 to 100 ng/ml, more preferably 10 to 50 ng/ml, most preferably about 20 ng/ml.

The growth factors may be added to the cells simultaneously or successively. The order of addition can be varied. It is preferred, however, that the growth factors are added to the cells simultaneously.

In one embodiment of the invention, at least one additional growth factor is added to the cells when inducing the differentiation into an endothelial phenotype. Optionally, at least two, at least three, or at least four additional growth factors are added to the cells for the induction of the endothelial phenotype. Examples of additional growth factors include but are not limited to insulin-like growth factor (IGF), epidermal growth factor (EGF), and the like.

In another embodiment, at least one inhibitor of 3-hydroxy-3-methylglutaryl-coenzyme A (HMG-CoA) such as Atorvastatin is added to the cells for the induction of the endothelial phenotype. The HMG-CoA inhibitor (e.g. Atorvastatin) may be added in combination with one or more growth factors or cytokines.

The cells can be cultured according to methods known in the art. They may be grown in plates or flasks coated with gelatin or collagen at a density of e.g. 5x10⁴ to 1x10⁵ cells/cm². The EPCs differentiate into (mature) endothelial cells due to the presence of the growth factors.

Usually, at least 10%, preferably at least 25%, more preferably at least 50%, even more preferably at least 75%, most preferably at least 90% of the cells in the population of endothelial cells express the marker protein CD144. Usually, at least 10%, preferably at least 25%, more preferably at least 50%, even more preferably at least 75%, most preferably at least 90% of the cells in the population of endothelial cells express the marker protein CD146. Usually, at least 10%, preferably at least 25%, more preferably at least 50%, even more preferably at least 75%, most preferably at least 90% of the cells in the population of endothelial cells express the marker protein CD31. Usually, at least 10%, preferably at least 25%, more preferably at least 50%, even more preferably at least 75%, most preferably at least 90% of the cells in the population of endothelial cells express the marker protein LDL-receptor. The endothelial cells in the population of cells preferably are capable of forming tubes in a Matrigel assay as described in the examples. In a preferred embodiment, the population of endothelial cells is characterized by a substantially uniform expression of the marker molecules CD144 (VE-cadherin), CD146, CD31 and/or LDL-receptor. More preferably, two, three, or all of CD144, CD 146, CD31 and LDL-receptor are expressed in a substantially uniform manner. The endothelial cells of the invention are further characterized by expression of VWF, which is secreted. When the cells express factor VIII, the molar ratio of VWF:FVIII in the supernatant can vary between 1:1 and 100:1. Furthermore, the cells may be capable of binding *Ulex europaeus* agglutinin. Usually, at least 10%, preferably at least 25%, more preferably at least 50%, even more preferably at least 75%, most preferably at least 90% of the cells in the population of endothelial cells express VWF. Usually, at least 10%, preferably at least 25%, more preferably at least 50%, even more preferably at least 75%, most preferably at least 90% of the cells in the population of endothelial cells are capable of binding *Ulex europaeus* agglutinin.

The endothelial cells of the invention preferably do not express the marker molecules CD45, CD14, CD133 and/or HLA-DR. Most preferably, none of the four marker molecules is expressed in the cells according to the invention. A fraction of the cells may express the marker CD34, e.g. 1 to 90%, preferably 5 to 50% of the cells.

In a specific embodiment, less than 90% of the cells express KDR (= VEGF receptor-2 = flk-1). Alternatively, less than 50% or less than 25% or less than 10% of the cells express KDR. In another embodiment, more than 90% of the cells express KDR.

The various embodiments concerning the expression of markers as described supra may be combined.

In a further step the endothelial cells are transduced with DNA encoding a therapeutically effective protein, e.g. a blood coagulation factor. Examples of therapeutically effective proteins include but are not limited to 1. blood coagulation factors, 2. growth factors (e.g. NEGF, FGF, SCF), 3. chemokines. The DNA may encode blood coagulation factor VIII, IX, or the like. Preferably, the DNA encodes human factor VIII. The DNA may code for mature wild type factor VIII or a mutein thereof. The factor VIII mutein may be a mutein having point mutations, a mutein being truncated at its C- or N-terminus, and/or a mutein partially or entirely lacking its B-domain.

US 6,346,513, WO 86/06101, WO 92/16557, and EP 0 123 945 describe deletions of the sequence coding for the B domain of factor VIII. EP 1 233 064, US 2002/0165177 and US 6,271,025 describe the insertion of introns into the cDNA encoding factor VIII. US 5,422,260 describes point mutations in the DNA sequence encoding factor VIII. The modifications in the coding or non-coding DNA sequence described therein can be used in accordance with the present invention. Also combinations of the modifications described may be employed. US 6,228,620, US 5,789,203 and US 5,693,499 describe the coexpression of DNA coding for the heavy chain and DNA coding for the light chain of factor VIII. These embodiments may also be used in accordance with the present invention.

In a particular embodiment, the factor VIII mutein has at least one of the following mutations (EP 1 136 553 A1):
- valine at position 162 is replaced by another neutral amino acid residue;
- serine at position 2011 is replaced by another hydrophilic amino acid residue;
- valine at position 2223 is replaced by an acidic amino acid residue;
- the B-domain between positions arginine 740 and glutamic acid 1640 is replaced by an arginine-rich linker peptide comprising 10 to 25, preferably 14 to 20 amino acid residues.

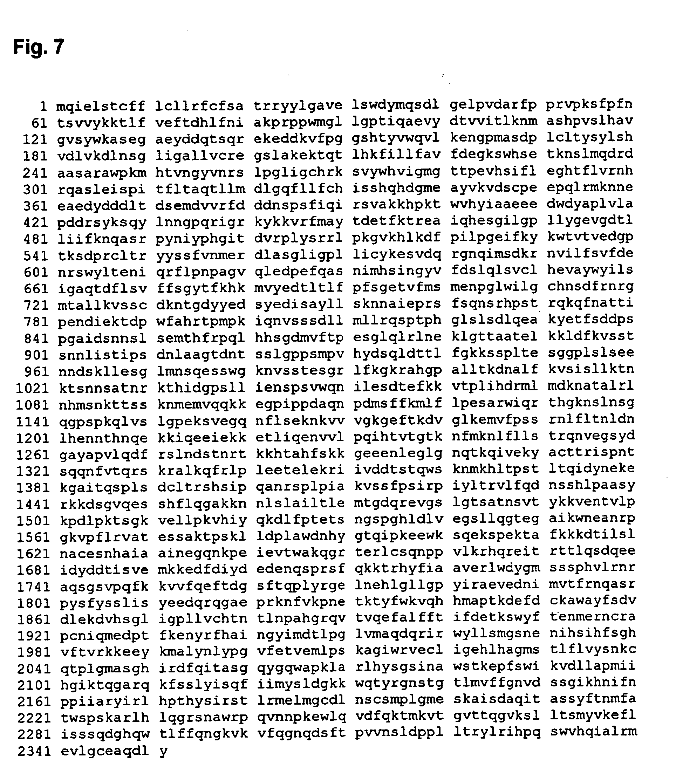

The positions refer to the published amino acid sequence of mature human factor VIII (Toole et al., Nature 1984, 312(5992):342-7; Wood et al., Nature 1984, 312(5992):330-7; Gitschier et al., Nature 1984, 312(5992):326-30). The amino acid sequence of human factor VIII is shown in figure 7 (SEQ ID NO:3). In another embodiment, the DNA encoding factor VIII is a modified factor VIII cDNA, wherein at least one intron has been inserted into at least one location of factor VIII cDNA.

The DNA encoding factor VIII is usually part of a vector that is used for transducing cells. The vector may comprise a promoter operably linked to the DNA sequence coding for the blood coagulation factor. It is preferred that the vector is a viral vector, preferably a retroviral vector, more preferably a lentiviral vector. More preferably, the lentiviral vector is an HIV-1-based vector. Besides HIV-1, lentiviral vectors based on HIV-2, simian immunodeficiency virus, equine infectious anemia virus, feline immunodeficiency virus (FIV) and visna virus may be used. In another embodiment the FVM encoding vector may also be a plasmid DNA (e.g. pcDNA3).

The endothelial cells of the invention can be cryopreserved and then be thawed and returned to culture without significant loss of their capacity to proliferate. To cryopreserve the cells, the culture cells can be detached and resuspended in suitable cryopreservation medium, i.e., containing a cryopreservation agent such as sugar(s), BSA, dimethyl sulfoxide (DMSO), glycerol, glycerol esters and the like.

In accordance with the present invention the cells may be frozen and thawed prior to transduction with DNA or after transduction with DNA. In another aspect of the invention, the cells are transduced prior to differentiating them from EPCs into mature endothelial cells.

In one embodiment, the transduced endothelial cells may be expanded by 4 to 10 orders of magnitude (10⁴ to 10¹⁰-fold), preferably by 5 to 9 orders of magnitude (10⁵ to 10⁹-fold). This expansion or proliferation step may be carried out after completion of steps a) and b). The expansion step is preferably carried out in vitro.

The endothelial cells described above are useful in a method for the production of a blood coagulation factor. The method comprises a) obtaining a population of human endothelial cells by a method according to any one of claims 1-5; and optionally generating an immortalized cell line from said population of human endothelial cells; b) culturing said population of human endothelial cells or said cell line under suitable conditions; and c) isolating the blood coagulation factor from the cell culture medium. The isolation step may comprise purifying the blood coagulation factor from the medium. Suitable purification steps include, but are not limited to, immunoaffinity chromatography, anion exchange chromatography, etc., and combinations thereof. Detailed purification protocols for coagulation factors from human blood plasma are, e.g., disclosed in WO 93/15105, EP 0 813 597, WO 96/40883 and WO 96/15140/50. They can be adapted to the requirements needed to isolate recombinant factors VIII and IX. For factor IX an effective protocol has been introduced containing an ammonium sulfate precipitation step followed by DEAE and HIC tentacle chromatography as well as heparin affinity chromatography (US 5,919,909). Quantity and activity of the purified protein during and after the purification procedure may be monitored by ELISA and coagulation assays.

Preferably, the blood coagulation factor is human factor VIII. In this case, factor VIII may be purified in the presence of VWF. The VWF may be present in the cell culture medium and is preferably used in an amount of 1 to 100, more preferably 50 to 60 mol VWF per mol factor VIII.

in case of the production of factor IX, the culturing is preferably performed in the presence of vitamin K which may be present in an amount of 0.1 to 100 µg/ml culture broth, more preferably 1 to 20 µg/ml culture broth.

A composition obtained by the method for the production of a protein, e.g., a blood coagulation factor can be subjected to virus inactivation treatment. A virus inactivation treatment includes heat treatment (dry or in liquid state, with or without the addition of chemical substances including protease inhibitors). After virus inactivation a further purifying step for removing the chemical substances may be necessary. In particular, for factor VIII isolated from blood plasma the recovery of a high purity virus-inactivated protein by anion exchange chromatography was described (WO 93/15105). In addition several processes for the production of high-purity, non-infectious coagulation factors from blood plasma or other biological sources have been reported. Lipid coated viruses are effectively inactivated by treating the potentially infectious material with a hydrophobic phase forming a two-phase system, from which the water-insoluble part is subsequently removed. A further advantage has been proven to complement the hydrophobic phase treatment simultaneously or sequentially with a treatment with non-ionic biocompatible detergents and dialkyl or trialkyl phosphates (WO 96/36369, EP 0 131 740, US 6,007,979). Non-lipid coated viruses require inactivation protocols consisting in treatment with non-ionic detergents followed by a heating step (60-65°C) for several hours (WO 94/17834).

A pharmaceutical composition comprising an effective amount of the isolated protein, e.g., the coagulation factor may further comprise pharmaceutically acceptable additives including human serum albumin (HSA; preferably about 1 mg/ml solution); inorganic salts such as CaCl₂ (preferably 2 to 5 mM), amino acids such as glycine, lysine, and histidine (preferably 0.1 to 1 M per amino acid); disaccharides such as sucrose and/or trehalose (preferably 0.4 to 1 M); organic salts such as Na-citrate (preferably up to 50 mM); etc. The preparations may be aqueous or non-aqueous. In the latter case the major component is glycerol and/or polyethylene glycol (e.g., PEG-300). The preparation may also be in the dry form (to be dissolved in the desired solvent prior to administration).

Another aspect of the invention is the use of a population of human endothelial cells for the preparation of a medicament for the treatment of hemophilia A or B, wherein said use comprises obtaining said population of human endothelial cells by a method according to any one of claims 1-5. The cells expressing factor VIII can be used in an ex vivo gene therapy protocol for hemophilia A. The transduced endothelial cells expressing factor VIII or IX may be transferred into an individual suffering from hemophilia A or B. Transfer methods include, but are not limited to the transplantation of synthetic vessels or prosthetic valves lined with transduced cells or the transplantation of a device or matrix designed to house transduced endothelial cells. The cells may also be introduced into the blood stream of a patient by conventional means such as intravenous infusion over a period of time.

Further described herein is the use of a population of cells according to the invention for the preparation of a medicament for the treatment of disorders of the cardiovascular system. These disorders include, but are not limited to, cardiac failure, acute and chronic heart disease. According to this aspect, the cells preferably provide a depot of autologous cells for the ectopic synthesis and secretion of any protein with systemic effect via the circulation. A particular aspect is revascularization in acute and chronic heart disease.

Usually, the cells obtainable according to the invention show a better therapeutic effect, since they may integrate into affected tissue in an improved manner. Also the homing of these cells may be significantly improved.

The cells obtainable according to the invention can be expanded by 4 to 10 orders of magnitude, preferably by 5 to 9 orders of magnitude and have the capacity to secrete a protein, e.g. factor VIII, upon lentiviral transduction. Therefore, endothelial cells prepared in accordance with the present invention can be used in hemophilia A gene therapy. In a specific embodiment, the expansion of the cells is limited. Accordingly, the endothelial cells may cease to proliferate within 15 weeks, preferably within 12 weeks, optionally within 8 or 9 weeks, starting from the addition of at least one growth factor for differentiation into endothelial cells. Cells with limited proliferation potential are preferred for use in a gene therapy protocol.

Endothelial cells prepared in accordance with the present invention express high levels of factor VIII. The amount of factor VIII secreted by the cells is at least 2.5 IU/10⁶ cell/48 h, preferably at least 5 IU/10⁶ cell/48 h, most preferably at least 7 IU/10⁶ cell/48 h.

Further described herein is a method for preparing an immortalized cell line from the population of endothelial cells described herein. The method includes transformation of the endothelial cells. The cells may be converted to an immortalized cell line by viral and/or non-viral infection (e.g. human papilloma virus, Epstein-Barr virus, DNA-plasmids), transfer of genes coding for human telomerase submit proteins such as telomerase reverse transcriptase (hTERT) or of one or more oncogenes.

This embodiment is preferred for in vitro-production and subsequent isolation of blood coagulation factor, e.g., FVIII.

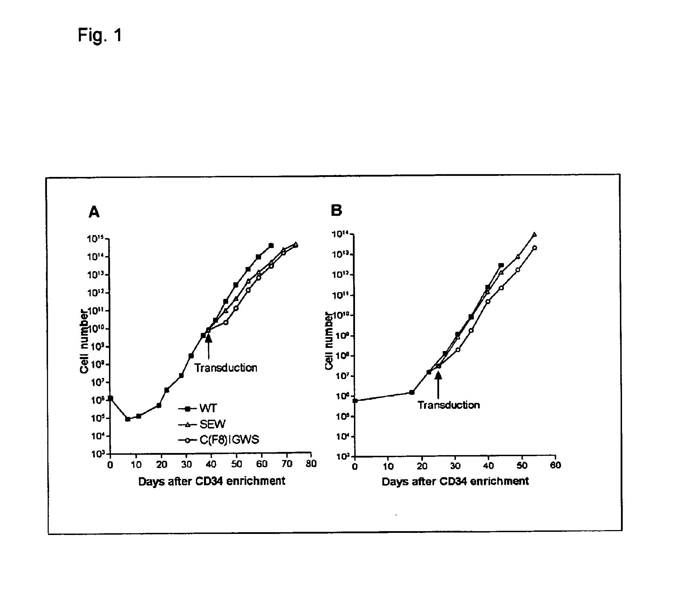

Fig. 1. Cumulative growth curves of endothelial cells. In the representative experiments shown here, about 10⁶ CD34-positive cells from cord blood obtained from two pools of donors were cultured in EDM on gelatin-coated plates. The adherent cells were then passaged and cultured in EGM-2 until senescence. The graph defined by the black squares (WT; ■) gives the number of endothelial cells plotted against the time after the first passage. On day 37 (A) and 23 (B), respectively, 10⁴ cells were plated for transduction 48 hours later with the construct cPPT-C(FVIIIΔB)IGWS (abbreviated as C(F8)IGWS; open circles; o) and pHR'SIN.cPPT-SEW (abbreviated as SEW; open triangles; Δ). These cells were also kept in EGM-2 until they ceased to proliferate.

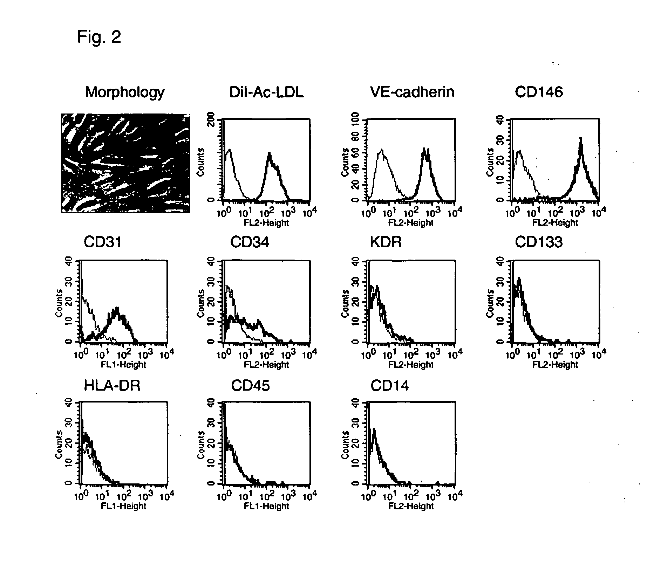

Fig. 2. Morphology and phenotypic characterisation of CBECs (cord blood-derived endothelial cells). CBECs from four different batches were analyzed for uptake of Dil-Ac-LDL and expression of cell surface markers by flow cytometry at various time points during culture. The x-axis gives the fluorescence intensity of the analyzed cells. Cells that were labelled with Dil-Ac-LDL or antibodies are represented by the thick lines, whereas the thin lines represent the appropriate negative controls. The panels show that CBECs were uniformly positive for the uptake of Dil-Ac-LDL and for the endothelial markers VE-cadherin, CD146 and CD31. Subsets of cells expressed CD34 and, albeit very weakly, KDR. All CBECs were negative for CD133, HLA-DR, CD45 and CD14.

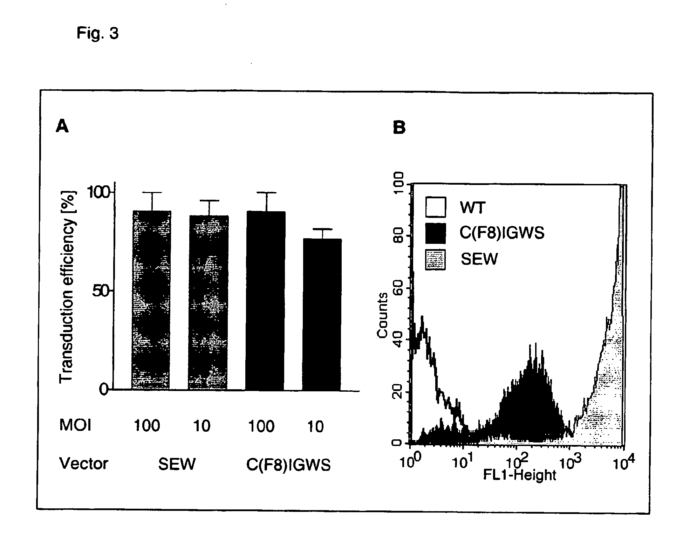

Fig. 3. Lentiviral transduction of endothelial cells. In four independent experiments, CBECs at passage numbers three to eight were transduced with the lentiviral constructs pHR'SIN.cPPT-SEW (abbreviated as SEW) and cPPT-C(FVIIIΔB)IGWS (abbreviated as C(F8)IGWS). (A) Transductions were performed with the constructs at MOls of 10 and 100 for each vector, and transduction efficiencies were determined by analysis of EGFP expression with untransduced cells as negative controls. In (B), the histogram illustrates the level of EGFP expression detected by FACS. The diagram shows the result of a representative example of four experiments. FACS analysis was performed 30 days after transduction with C(F8)IGWS (MOI 10; black area under the curve) and with SEW (MOI 10; grey area under the curve). Untransduced cells served as negative controls (white area under the curve).

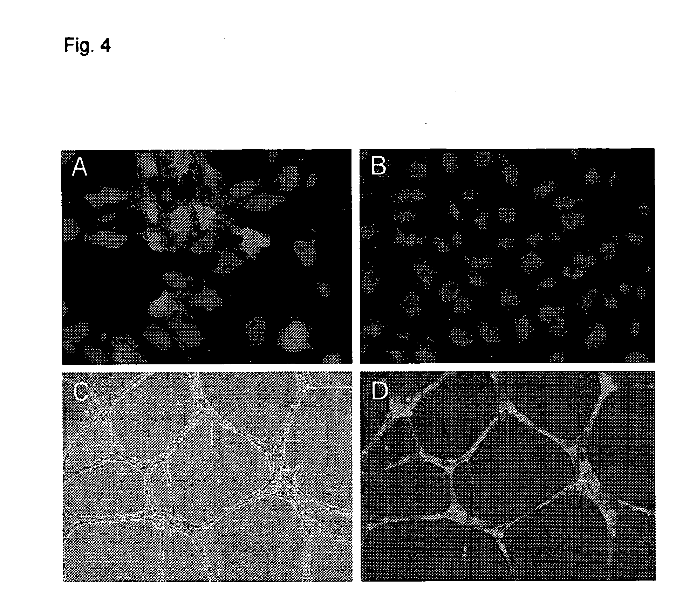

Fig. 4. Phenotype of transduced CBECs: Incorporation of Dil-Ac-LDL and *in vitro* tube formation. (A&B) Five weeks after transduction with pHR'SIN.cPPT-SEW (MOI 100), cells were incubated with Dil-Ac-LDL for one hour and examined for EGFP expression (A) and Dil-Ac-LDL uptake (B) by fluorescence microscopy. (C&D) For the tube formation assay, CBECs were detached by trypsin/EDTA treatment and plated on Matrigel^{®} basement membrane matrix. The cells were incubated at 37°C for 8 to 10 hours and examined microscopically for the formation of angiogenic tubes (C: phase contrast; D: EGFP expression).

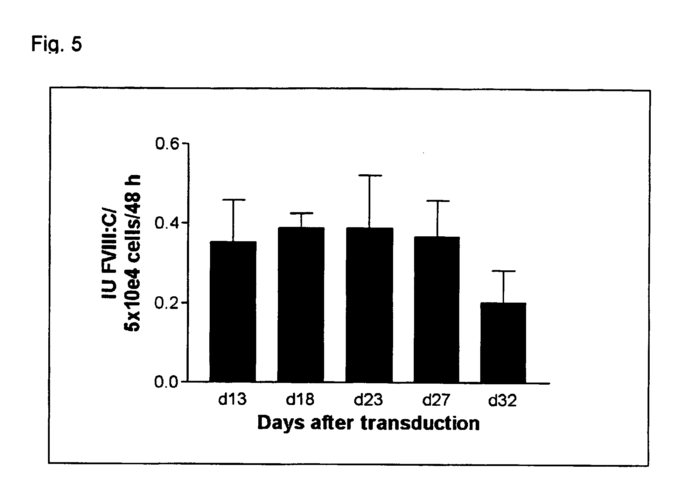

Fig. 5. Quantification of FVIII:C in CBEC supernatants. CBECs from three pools of donors were transduced with the BDD FVIII-encoding construct cPPT-C(FVIIIAB)IGWS at the MOI of 10. In order to quantify secretion of FVIII:C at various timepoints after transduction, 5x10⁴ cells were plated in 1 ml of EGM-2 and incubated at 37°C. After 48 hours, supernatant was harvested, cleared of cellular debris by short centrifugation and stored at -80°C until analysis. Every assay included supernatans from cells transduced with pHR'SIN.cPPT-SEW and from untransduced cells. No FVIII:C could be detected in any of these controls (detection limit 0.01 IU/ml).

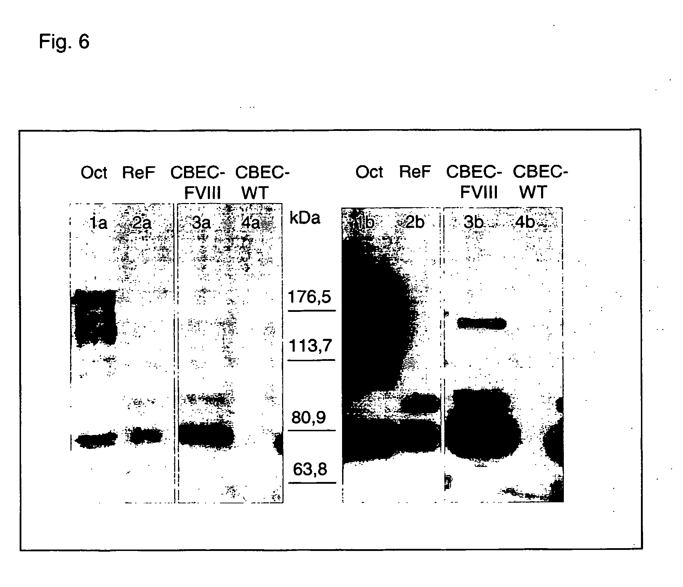

Fig. 6. Detection of FVIII protein in concentrated CBEC supernatants by immunoblotting. Serum-free culture supernatants from untransduced CBECs and from CBECs that were transduced with cPPT-C(FVIIIΔB)IGWS were concentrated 200 to 400fold by ultrafiltration. The samples were separated by SDS-PAGE and subjected to immunoblotting. Lanes 1 and 2 show commercially available FVIII concentrates as reference. Lanes 3 and 4 contain concentrated supernatant from CBECs. In order to detect signals of various intensities, the autoradiography film was exposed for different periods of time (1a-4a: one minute; 1b-4b: 20 minutes). Lane 1: Oct, Octanate^{®} (plasma-derived full-length FVIII); lane 2: ReF, ReFacto^{®} (recombinant BDD FVIII); lane 3: supernatant from cPPT-C(FVIIIΔB)IGWS transduced CBECs (BDD FVIII); lane 4: supernatant from untransduced CBECs.
Fig. 7 shows the amino acid sequence of human factor VIII (amino acids 20-2351 correspond to mature factor VIII).

The following non-limiting examples further illustrate the invention.

### 1. MATERIALS & METHODS

### ISOLATION OF CD34-POSITIVE CELLS FROM CORD BLOOD

Mononuclear cells were isolated by Ficoll density separation (d=1.077 g/ml) from cord blood from healthy donors and contained less than 2% CD34⁺ cells as determined by immunofluorescence staining and fluorescence-activated cells sorting (FACS) analysis. The cells were washed twice in Dulbecco's PBS (BioWhittaker, Verviers, Belgium) containing 2 mM EDTA (Sigma, Taufkirchen, Germany) and 0.5% human serum albumin (HSA; DRK-Blutspendedienst Niedersachsen, Springe, Germany), and CD34-positive cells were enriched by magnetic activated cell sorting (MACS) using the Direct CD34 Progenitor Cell Isolation Kit (Miltenyi Biotec, Bergisch Gladbach, Germany) according to the manufacturer's instructions. The purity of CD34⁺ cells was in the range of 80% to 95%.

### DIFFERENTIATION OF ENDOTHELIAL CELLS

CD34⁺ cells were resuspended in endothelial differentiation medium consisting of 80% basal lscove medium (Biochrom, Berlin, Germany), 10% horse serum (PAN Biotech, Aidenbach, Germany; selected lots), 10% heat-inactived fetal calf serum (FCS; Biochrom, selected lots), L-alanyl-L-glutamine (final concentration 2 mM; BioWhittaker) and penicillin-streptomycin (final concentrations 100 U/ml and 100 µg/ml, respectively; BioWhittaker). The medium was filtered (pore size 0.22 µm) and then supplemented with recombinant human (rh) VEGF (vascular endothelial growth factor; 50 ng/ml), rh basic FGF (fibroblast growth factor-2; 20 ng/ml), rh SCF (stem cell factor; 50 ng/ml; all from R&D Systems, Wiesbaden, Germany), and rh SCF- (stem cell growth factor β; 20 ng/ml; PeproTech, Frankfurt am Main, Germany). This medium including the growth factors will be referred to as endothelial differentiation medium (EDM).

CD34⁺ cells were cultured in tissue-culture treated 6-well plates coated with 1% gelatin solution (Sigma) at 5x10⁴-1x10⁵ cells/cm² in 3 ml per well at 37°C with 5% CO₂ in a humidified atmosphere. If the cell yield from one donor was lower than 5x10⁵, the cells were cultured in 24-well plates, or cells from different donors were pooled. Half of the medium was exchanged twice a week, and the cells were incubated until the wells were 80%-90% confluent with adherent cells (usually up to three weeks).

Adherent cells were detached by trypsin/EDTA treatment and then cultured on gelatin-coated tissue culture flasks in medium EGM-2 (endothelial cell basal medium (EBM)-2 supplemented with 2% FCS, VEGF, rh EGF (epidermal growth factor), rh basic FGF, R³-IGF-1 (insulin-like growth factor), hydrocortisone, ascorbic acid, heparin, gentamicin, and amphotericin B; Clonetics/BioWhittaker, Verviers, Belgium). Cells could be expanded most efficiently when grown at low density (1 x 10³/cm²).

### IMMUNOFLUORESCENCE

Approximately 10⁵ cells were prepared for flow cytometry by washing with PBS containing 1% FCS (Biochrom) and 0.1% sodium azide (Sigma). In order to characterize the expression of hematopoietic and endothelial cell surface markers, the following monoclonal antibodies (mAb) were used: anti-CD14-PE, anti-CD34-PE, anti-CD45-FITC, anti-HLA-DR-FITC (all from BD Biosciences, Heidelberg, Germany), anti-CD133-PE (Miltenyi Biotec), anti-CD146, anti-CD146-FITC, anti-VE-cadherin (all from Chemicon, Temecula, CA), anti-34-PC5 (Immunotech, Marseille, France), anti-CD31-FITC and anti-KDR (both from Sigma). Cells were incubated with the respective mAb for 20 minutes at 20°C (anti-CD31-FITC, anti-KDR) or at 4°C (all others). In a second step, unconjugated primary mAb were detected by incubation with RPE-conjugated F(ab')₂ fragment of rabbit anti-mouse immunoglobulin (Dako, Glostrup, Denmark). Samples of cells were also stained with isotype-matched control antibodies (purchased from BD Biosciences and Immunotech). FACS analyses were performed using a FACScan flow cytometre (BD Biosciences) and Cell Quest software (BD Biosciences). Each analysis included at least 10,000 events. Dead cells were excluded based on their forward scatter and side scatter properties.

### Dil-Ac-LDL LABELING

Fluorescent labeling of endothelial cells by uptake of acetylated low-density lipoprotein (Ac-LDL) was performed by incubating the cells with 2 µg/ml Dil-Ac-LDL (Harbor Bio-Products, Norwood, MA) in EGM-2 for 60 minutes at 37°C. Then the medium was exchanged for EGM-2, and the Dil-Ac-LDL uptake of the cells was analyzed by fluorescence microscopy (Nikon Eclipse TE300) and flow cytometry.

### LENTIVIRAL CONSTRUCTS

Two HIV-1-derived self-inactivating lentiviral gene transfer constructs were used in this example: The vector pHR'SIN.cPPT-SEW (Demaison *et al.,* 2002) contains a gene expression cassette consisting of the enhancer region of the spleen focus forming virus (U3-LTR), the cDNA of the enhanced green fluorescent protein (EGFP) and the Woodchuck hepatitis virus posttranscriptional regulatory element (WPRE).

In order to generate the vector cPPT-C(FVIIIAB)IGWS, three cloning steps were required. First, the HIV-1-based monocistronic self-inactivating vector pRRL-CMV-GFP-WPRE-SIN (CGWS, derivative of the vectors pRRL-PGK-GFP-SIN-18 and pHR'-CMV-lacZ-SIN-18 kindly provided by R. Zufferey, Geneva, Switzerland) encoding the EGFP gene as a marker gene flanked 5' by an internal CMV promoter and 3' by the WPRE sequence was modified by insertion of a multiple cloning site (MCS) and the internal ribosome entry site (IRES) of the encephalomyocarditis virus (ECMV; MCS and IRES from pIRES2-EGFP, Clontech, Heidelberg, Germany). In a second step, the central polypurine tract and central termination sequence (cPPT/CTS; Chameau *et al.,* 1992; Zennou *et al.,* 2000; Sirven *et al.,* 2000; Follenzi *et al.,* 2000) from HIV-1 was cloned into the Clal site 5' of the internal CMV promoter. The cPPT/CTS fragment flanked by Clal restriction sites was obtained by polymerase chain reaction (PCR) using the oligonucleotides PPTCTSCLA-5: 5'-CCA TCG ATA CAA ATG GCA TTC ATC C-3' (SEQ ID NO:1) and PPTCTSCLA-3: 5'-CCA TCG ATC TCG AGC CAA AGT GGA TCT CTG CTG TCC-3' (SEQ ID NO:2) with a plasmid containing the HIV-1 LAI gag-pol cDNA (Myers *et al*., 1989) as template. Finally, the cDNA for human B-domain deleted FVIII (huBDD FVIII; deletion as described in Tonn *et al.,* 2002) was cloned into the SaII site of the MCS between CMV promoter and IRES to yield the bicistronic vector cPPT-C(FVIIIΔB)IGWS.

### PRODUCTION OF LENTIVIRAL SUPERNATANTS

To generate lentiviral particles, gene transfer vector DNA was transiently introduced into 293T cells (cultured as decribed in Tonn *et al*., 2002) by triple co-transfection with the packaging construct pCMVAR8.91 (Zufferey *et al.,* 1997) encoding gag, pol, rev, and tat and the pseudotyping construct pMD2.VSVG (Follenzi and Naldini, 2002) coding for the vesicular stomatitis virus glycoprotein (VSV-G).

Transfection of plasmid DNA was performed by calcium phosphate coprecipitation. Sixteen hours after transfection, cells were given fresh medium (DMEM [Gibco, Karlsruhe, Germany] supplemented with 10% heat-inactivated FCS, 4 mM L-glutamine, and penicillin-streptomycin at 100 U/ml and 100 µg/ml, respectively). After further 24 hours, the viral supernatant was collected, filtered (pore size 0.22 µm), concentrated by ultracentrifugation and filtered again. Virus titers were determined as 293T-transducing units (TU/ml) by transduction of 293T cells with dilutions of vector concentrate and subsequent FACS analysis. Concentrated vector stocks of the constructs pHR'SIN.cPPT-SEW and cPPT-C(FVIIIAB)IGWS had titers between 10⁸ and 10⁹ and between 10⁷ and 10⁸ TU/ml, respectively.

### TRANSDUCTION OF ENDOTHELIAL CELLS

Endothelial cells were plated on gelatin-coated 6-swell plates at 1 x 10⁴ cells per well in EGM-2 and incubated at 37°C for 48 hours. For the transduction, the medium was exchanged for unconcentrated or concentrated virus supernatant in EGM-2 in the presence of 4 µg/ml protamine sulfate (Sigma) and 50 µM dNTPs (New England Biolabs, Frankfurt am Main, Germany). After spinoculation (1250g, 90 minutes, 32°C), the cells were incubated for further 16 hours at 37°C. Then the virus supernatant was removed and the cells were given fresh EGM-2. The multiplicities of infection (MOIs) were calculated as ratios of 293T-TU/ml to target cells. EGFP expression was analyzed by FACS analysis and fluorescence microscopy at various time points.

### FVIII QUANTIFICATION

To assess the amount of secreted FVIII, transduced endothelial cells and untransduced control cells were seeded at 5 x 10⁴ cells in a total volume of 1 ml EGM-2 in gelatin-coated 12-well plates. After 48 hours, the supernatants were cleared of cellular debris by centrifugation (700g, 3 minutes, 4°C) and stored in multiple aliquots at -80°C until analysis. FVIII antigen (FVIII:Ag) and FVIII activity (FVIII:C) were determined with the commercially available Immunozym FVIII:Ag ELISA and the Immunochrom FVIII:C chromogenic assay respectively (Immuno, Heidelberg, Germany) according to the manufacturer's instructions. FVIII levels are given as international units (IU)/ml with 150 ng/ml corresponding to 1.0 IU/ml. FVIII standards in both assays were calibrated against WHO plasma standards by the manufacturer.

### FVIII WESTERN BLOT

Confluent layers of endothelial cells were washed with PBS and cultured with EGM-2 without FCS for 15-24 hours. Culture supernatants were filtered (0.22 µm) to remove cellular debris and concentrated up to 400fold by ultracentrifugation in Vivaspin 20 concentrators (Sartorius, Göttingen, Germany) with a molecular weight cut-off (MWCO) of 30 kDa. The concentrates were boiled for 5 minutes in Laemmli buffer (RotiLoad 1; Roth, Karlsruhe, Germany), separated by SDS-PAGE on 10% polyacrylamide gels and transferred to a polyvinylidine difluoride membrane (Roth). The membranes were blocked for 3 hours at 20°C in 5% powdered skim milk and incubated at 4°C for 15-20 hours with polyclonal sheep anti-human factor VIII:C antibody (Enzyme research Laboratories, Swansea, UK). After extensive washing, the blots were incubated with a peroxidase-conjugated donkey anti-sheep IgG secondary antibody (Sigma) for 40 minutes at room temperature. After further washing, the proteins were visualized by enhanced chemiluminescence (Pierce, Bonn, Germany). As positive controls, we used recombinant B-domain deleted FVIII ReFacto^{®} (Pharmacia & Upjohn, Martinsried, Germany) and plasma-derived human FVIII (Octanate^{®}; kindly provided by Lothar Biesert, Octapharma, Frankfurt am Main, Germany).

### IN VITRO MATRIGEL ASSAY

Prechilled 24-well plates were coated with 500 µl Matrigel^{®} basement membrane matrix (BD Biosciences) per well and incubated for 1 hour at 37°C. Endothelial cells were harvested by trypsin/EDTA treatment, resuspended in EGM-2, and seeded on top of the gelled Matrigel^{®} at 6 x 10⁴ to 1 x 10⁵ cells in 400 µl. Cultures were incubated at 37°C. After 8 to 10 hours, the cultures were checked for tube formation by phase contrast and fluorescence microscopy (Nikon Eclipse TE300).

### STATISTICAL ANALYSIS

Data are presented as means ± SD. The paired Student's t-test was used to compare transduction efficiencies and FVIII secretion levels of cells transduced at different multiplicities of infection (MOIs). Statistical analysis was performed using the GraphPad Prism 3.0 software.

### 2. RESULTS

### DIFFERENTIATION AND CULTURE OF CORD BLOOD-DERIVED ENDOTHELIAL CELLS (CBECs)

After isolation of cord blood mononuclear cells, CD34⁺ cell fractions with a purity of 80% to 95% were obtained using MACS immunomagnetic beads. Cells from single donors or cell pools from several donors were cultured in EDM containing rh VEGF, rh basic FGF, rh SCF and rh SCGF-β (see Materials & Methods) for about three weeks. When proliferating adherent cells with endothelial morphology could be detected, they were passaged before reaching confluency. With an input of 10⁵ to 10⁷ CD34⁺ cells, adherent cells could be expanded up to 10⁹ fold during a total culture time of eight weeks. Fig. 1 shows the cumultative growth curves of two representative experiments with cells from donor pools (denoted as 'WT'). In this example, cells proliferated with a doubling time of about 30-35 hours and were expanded up to more than 10⁸ fold before they ceased to grow and the culture became senescent.

Cultures with mononuclear cells from CB prior to CD34 isolation and with the CD34-depleted fraction either did not yield any detectable EC differentiation under otherwise identical conditions or yielded a considerably lower cell number. Therefore, the enrichment of CD34-expressing cells is preferred. The data suggest that the EC were derived from CD34⁺ progenitor cells.

### PHENOTYPIC CHARACTERISATION OF CBECs

The adherent cells grew as monolayers of spindle-shaped flat cells (Fig. 2). The cells were able to incorporate Dil-Ac-LDL which was detected by fluorescence microscopy and flow cytometry (Fig. 2) and showed formation of tubuli in the Matrigel assay (see below). The cells were further characterized by flow cytometry with respect to the expression of various endothelial and hematopoietic surface markers and found to be uniformly positive for VE-cadherin (CD144), CD146 and CD31, which are typical of endothelial cells. The cells were uniformly negative for the hematopoietic markers CD45, CD14, CD133 and HLA-DR (Fig. 2). FACS analysis of five batches of EC showed that the cells obtained with this protocol were heterogenous concerning the expression of CD34 and KDR/VEGF-R2 (Fig. 2). CD34 expression was detected on a subset of 5-45% of cells, whereas KDR was weakly expressed by less than 5% of CBECs. The expression of the aforementioned cell surface markers appeared to be unchanged throughout cell expansion.

The data concerning expression of VE-cadherin, CD146, CD31, CD45, CD34 and KDR were confirmed by immunohistochemical analysis (IHC). Additionally, the expression of the endothelial marker von Willebrand factor (vWF) and and binding of *Ulex europaeus* agglutinin could be demonstrated by IHC.

### TRANSDUCTION EFFICIENCY

In order to see whether CBECs can be transduced efficiently with lentiviral vectors, CBECs from four different pools of donors were transduced in a first series of experiments with the constructs pHR'SIN.cPPT-SEW encoding EGFP and cPPT-C(FVIIIΔB)IGWS encoding huBDD FVIII and EGFP at passage numbers three to eight and at multiplicities of infection

(MOIs) of 10 and 100. The transduction efficiency was determined by detection of EGFP-positive cells at least at two different time points and not earlier than eight days after transduction.

For the transduction of EC, MOIs of 10 yielded transduction efficiencies of 88.2%±7.6% (range 80.3%-95.6%) and 76.9%±5.0% (range 73.1%-84.2%) with pHR'SIN.cPPT-SEW and cPPT-C(FVIIIOB)IGWS, respectively (Fig. 3A). When the MOIs were raised to 100, there was no statistically significant increase in transduction efficiencies (90.4%±9.5% and 90.6%±9.7%. p>0.05 for both vectors; Fig. 3A).

The percentage of EGFP-expressing cells as well as the EGFP expression level remained relatively unchanged during the culture period. Fig. 3B shows the EGFP expression of transduced vs. non-transduced cells 30 days after transduction with MOIs of 10 in a representative experiment.

### INFLUENCE OF LENTIVIRAL TRANSDUCTION ON CELL PHENOTYPE AND CELL PROLIFERATION

We observed some vector-mediated toxicity on the transduced EC which was apparent due to cell death during/after transduction (in particular with the higher MOI) and a lag phase before the cells returned to the proliferation rate of untransduced control cells as illustrated in Fig. 1. Hence, we wanted to investigate whether this was accompanied by alterations of cell phenotype between the transduced cells and the control cells. Cells were analyzed several weeks after transduction for the expression of CD146, CD34, KDR, and CD133 by flow cytometry and for the capability to incorporate Dil-Ac-LDL. The expression of the above mentioned cell surface markers was unchanged in comparison to untransduced cells, as was the uptake of Dil-Ac-LDL (Fig. 4A&B). In addition, the CBECs retained their ability to form tubes in the Matrigel assay (Fig. 4C&D) which is characteristic of mature and functional EC.

### QUANTIFICATION OF FVIII SECRETION BY CHROMOGENIC ASSAY AND ELISA

The capacity of CBECs to express huBDD FVIII was investigated in seven independent experiments. Cells were transduced with the lentiviral construct cPPT-C(FVIIIAB)IGWS encoding huBDD FVIII and EGFP and cultured as described above until senescence. FVIII secretion was quantified at various time points by seeding 5x10⁴ cells in 1 ml EGM-2 in 12 well plates and collecting the cell culture supernatants after 48 hours. Aliquots were stored at -80°C until analysis using a chromogenic assay and an ELISA with a monoclonal antibody against the light chain as detection antibody. Cells were maintained from the transduction until they ceased to proliferate.

FVIII:C levels as measured by chromogenic assay were relatively constant until senescence. During the first four weeks after transduction of cells from three pools of donors, mean FVIII:C levels were 0.35-0,39 IU/5x10⁴ cells/48 h at an MOI of 10 corresponding to 7,0-7,8 IU/10⁶ cells/48 h (Fig. 5). FVIII:C secretion then decreased in all cultures which was accompanied by reduced proliferation and finally senescence. In a second set of experiments, the influence of the MOI on FVIII secretion was investigated. Raising the MOI from 10 to 100 lead only to a slight increase in FVIII:C secretion which was not statistically significant (p>0.05, n=4). Cells that were transduced with the control construct pHR'SIN.cPPT-SEW and untransduced cells did not secrete detectable amounts of FVIII:C (both <0.01 IU/5x10⁴ cells/48 h; n=7).

Determination of FVIII:Ag levels in the same EC supernatants yielded slightly higher FVIII:Ag concentrations with 0.45-0,66 IU/5x10⁴ cells/48 h at an MOI of 10 compared to FVIII:C (n=3). This resulted in mean ratios of FVIII:C/FVIII:Ag of 0.54-0,83 (n=3; Table 1). In a control experiment, human umbilical vein endothelial cells (HUVECs) were transduced with the same huBDD FVIII-encoding vector. In six supernatants, the ratio of FVIII:C/FVIII:Ag was found to be 0.91±0.23. The latter ratio does not differ from the ratios that are characteristic of several hematopoietic cell lines transduced with a FVIII-encoding vector containing the same transgene cassette (Tonn *et al.,* 2002).

Table 1. Specific activity of recombinant BDD FVIII in different human cell types. Hematopoietic cell lines, HUVECs and CBECs (cord-blood derived endothelial cells) were transduced with lentiviral vectors containing the same FVIII expression cassette. The specific activity was calculated as ratio between FVIII:Ag and FVIII:C in the supernatant of these cells and is given as mean values of at least three experiments.

| **Transduced cell type** | **Mean ratio FVIII:C/FVIII:Ag** |
|---|---|
| CBECs (Cord blood CD34⁺-derived endothelial cells) | 0,54-0,83 |
| HUVECs | 0,91 |
| Hematopoetic cell lines (Tonn *et al.,* 2002) | 0,85-1,02 |

### CHARACTERISATION OF SECRETED FVIII BY WESTERN BLOT ANALYSIS

in order to confirm the assumption that EC secrete correctly processed and hence active procoagulant FVIII, FVIII was enriched from cell culture supernatants by ultrafiltration using Vivaspin concentrators with a molecular weight cut-off of 30 kDa. Since the serum content of the EGM-2 did not allow effective concentration, EC were cultured over night without serum. Samples were concentrated up to 400fold and analyzed by ELISA, FVIII Western blotting and chromogenic assay.

Concentrated supernatants of EC transduced with cPPT-C(FVIIIAB)IGWS and of untransduced cells were subjected to SDS-PAGE and immunoblotting. As reference, plasma-derived FVIII (Octanate^{®}) and recombinant B-domain deleted FVIII (ReFacto^{®}) were used. Octanate^{®} consists of a doublet of light chains of about 80 kDa and heavy chains of various sizes due to the different proteolytic steps involving the B domain *in vivo.* ReFacto^{®} is composed of an 80 kDa doublet of light chains and a 90 kDa heavy chain. The deletion of the B domain used to express ReFacto^{®} is almost identical to the deletion in the gene transfer vector that we used. Using a polyclonal anti-FVIII antibody, we therefore expected bands for the light chain at the same height in the EC-derived FVIII and the reference preparations and also comparable sizes of the heavy chain in EC-derived FVIII and ReFacto^{®}. Fig. 6 demonstrates that this was indeed the case. After longer exposure, a weak band at 170 kDa appeared which indicates that the intracellular cleavage of FVIII into heavy chain and light chain might not have been complete. No FVIII protein could be detected in concentrated supernatants of untransduced cells.

Before immunoblotting, the FVIII:Ag concentrations of the EC-derived samples were determined by ELISA. The signal intensity found for EC-derived FVIII is in good agreement with the reference samples. With a chromogenic assay, we also confirmed that the concentrated supernatants exhibited procoagulant activity.

The invention provides a novel combination of cytokines for the differentiation of endothelial cells from CD34-positive cells, and it has been shown that umbilical cord blood was a feasible source of cells. After enrichment of CD34-positive cells from cord blood, the cultures containing VEGF, FGF-2, SCF and SCGF-β yielded adherent cells that had an endothelial phenotype and a very high, but limited proliferative potential. Previous studies have shown that endothelial cells can be derived and expanded considerably from progenitor cells in peripheral blood (Gehling *et al.,* 2000; Lin *et al.,* 2000) and bone marrow

(Quirici *et al.,* 2001), but umbilical cord blood has not been investigated in this respect. In the present study, the uniform expression of VE-cadherin (CD144), CD31, CD146 and LDL receptor, the absence of expression of CD45 and CD14, and the tube formation in the Matrigel assay demonstrated a clearly endothelial phenotype. A substantial subset of the cells retained the expression of CD34, whereas a surprisingly small percentage was only weakly KDR-positive. This pattern of cell surface markers has not been reported yet and differs from the studies of Gehling *et al.* (2000) and Lin *et al.* (2000) with respect to the expression of CD34, KDR, CD144 and CD31. The cells obtained with Gehling's protocol from CD133⁺ cells from G-CSF-mobilized peripheral blood were almost uniformly KDR-positive with only subsets expressing CD31, CD34 and CD144. Blood outgrowth endothelial cells (BOECs) from peripheral blood mononuclear cells that were differentiated according to Lin's protocol were uniformly positive for CD34, KDR, CD144 and CD31. The Matrigel assay has not been included in the other studies so that the cells cannot be compared with respect to the functional feature of tube formation. The cells also differed in their proliferative potential, which appeared to be huge in Lin's protocol (18 logs within 60 days) and relatively low in Gehling's study, whereas the protocol described supra allows for an intermediate expansion of cultures by 5 to 9 logs. After a total *ex vivo* culture period of several months with a constant cell phenotype, eventually senescence of cultures could be observed. Since excessive and uncontrollable proliferation *in vivo* as reported by Lin and coworkers on BOECs in immunodeficient mice might entail serious adverse effects, the limited expansion potential of the endothelial cells presented herein might actually be advantageous.

CBECs could also be useful target cells for hemophilia A gene therapy. A stably integrating, HIV-1-derived self-inactivating lentiviral vector with a B-domain deleted factor VIII cDNA and EGFP as marker gene was used for the transduction experiments. A moderate MOI of 10 proved to be sufficient to achieve high rates of transduction that were typically in the range of 75% to 95% for the FVIII/EGFP- or the EGFP-control construct. The transductions resulted in very high levels of FVIII secretion that were determined by a chromogenic assay and by ELISA. FVIII:C levels corresponded to 7,0-7,8 IU/10⁶ cells/48h and were among the highest levels reported so far in other recombinant systems. Comparable levels of FVIII secretion were achieved in transduced HUVEC with the same vector, but 10-20fold lower levels in hematopoietic cell lines that were transduced with the same transgene cassette (Tonn *et al.,* 2002). In addition, the level of FVIII secretion in HUVEC was found to be more than 10fold higher than reported by Chuah and colleagues who used a retroviral vector (Chuah *et al.,* 1995).

The data on FVIII secretion were confirmed by a FVIII-specific ELISA, which yielded even slightly higher FVIII:Ag values so that the mean ratio of FVIII:C/FVIII:Ag was in the range of 0,54-0,83 (instead of about 1 as for hematopoietic cell lines and HUVEC). This reduction in specific activity could be caused by incomplete intracellular proteolysis because Western blotting of concentrated cell supernatant demonstrated that a part of the FVIII protein was secreted as a 170 kDa precursor with most of the FVIII being processed into heavy and light chains of 90 and 80 kDa as expected. But since FVIII activation in the plasma involves further proteolytic steps, the procoagulant activity *in vivo* of the CBEC-derived FVIII would probably be in the normal range.

The examples indicate that endothelial cells are particularly suited for the recombinant expression of FVIII and that the lentiviral vector allows for considerably more efficient recombinant FVIII expression than previously used viral or nonviral expression vectors. Although transduction experiments at an MOI of 10 resulted in some cell death during/after incubation with vector and a short lag phase in proliferation compared to untransduced cells, phenotypical alterations or a net reduction or increase of expansion of the transduced CBECs could not be detected. The levels of transgene expression as determined by a FVIII:C-specific chromogenic assay or by flow cytometry for EGFP remained relatively stable until the cells ceased to proliferate. Hence, the vector system chosen in the examples was not only highly efficient, but also safe in this preliminary *in vitro* analysis.

Endothelial cells derived from cord blood are an attractive autologous source of cells for hemophilia A gene therapy. It is known that for about two thirds of all hemophilia A patients, the underlying mutation is inherited and therefore their FVIII deficiency is predictable based on the family history. In these cases, the CD34-positive cells from umbilical cord blood that would usually be discarded could be used for endothelial differentiation cultures. For adolescent or adult patients, the protocol presented in the examples for CBECs can be adapted to CD34-positive cells from peripheral blood or bone marrow.

The invention therefore also relates to a method for the preparation of endothelial cells expressing a protein, e.g. a blood coagulation factor, comprising a) contacting in vitro human endothelial precursor cells with the growth factors VEGF, bFGF, SCF and SCGF-β; and b) transducing the cells with DNA encoding the protein. The endothelial precursor cells may be derived from bone marrow, peripheral blood or cord blood. The preferred embodiments of this method correspond to the preferred embodiments of the methods of the invention described supra.

After lentiviral transduction at an early time-point, the culture could be expanded and analyzed for efficient FVIII secretion and safety features such as the absence of replication-competent lentivirus. Frozen batches of cells could be stored and used for multiple injections during life.

The senescence observed in all *in vitro* cultures so far suggest a moderate number of population doublings *in vivo* and therefore the requirement for repeated cell infusions. This scenario might be preferable to the *in vivo* proliferation of FVIII-transfected BOECs that were shown to engraft in the spleen and in bone marrow of immunodeficient mice after injections into the tail vein without accompanying conditioning regimen (Lin *et al.,* 2002). On the basis of the data published so far, long-term complications such as replacement of bone marrow by BOECs leading to hematological abnormalities or enhanced risk of thrombosis will have to be considered and must be ruled out before any therapeutic application of these cells unless their *in vivo* proliferation can be controlled.

The present invention provides the use of endothelial cells, in particular CBECs for the gene therapy of hemophilia A and other congenital disorders that are characterized by the absence of plasma proteins such as factor IX, von Willebrand factor or α₁-antitrypsin.

### REFERENCES

ANDREWS, J.L., WEAVER, L., KALEKO, M., CONNELLY, S. (1999). Efficient adenoviral vector transduction and expression of functional human factor VIII in cultured primary human hepatocytes. Haemophilia 5, 160-168.
ASAHARA, T., MUROHARA, T., SULLIVAN, A., SILVER, M., VAN DER ZEE, R., Ll, T., WITZENBICHLER, B., SCHATTEMAN, G., ISNER, J.M. (1997). Isolation of putative progenitor endothelial cells for angiogenesis. Science 275, 964-967.
ASAHARA, T., ISNER, J.M. (2002). Endothelial progenitor cells for vascular regeneration. J. Hematoth. Stem Cell Res. 11, 171-178.
CHARNEAU, P., ALIZON, M., CLAVEL, F. (1992). A second origin of DNA plus-strand synthesis is required for optimal human immunodeficiency virus replication. J. Virol. 66, 2814-2820.
CHUAH, M.K.L., VANDENDRIESSCHE, T., MORGAN, R.A. (1995). Development and analysis of retroviral vectors expressing human factor VIII as a potential gene therapy for hemophilia A. Hum. Gene Ther. 6, 1363-1377.
CHUAH, M.K.L., BREMS, H., VANSLEMBROUCK, V., COLLEN, D., VANDENDRIESSCHE, T. (1998). Bone marrow stromal cells as targets for gene therapy of hemophilia A. Hum. Gene Ther. 9, 353-365.
CHUAH, M.K.L., COLLEN, D., VANDENDRIESSCHE, T. (2001). Gene therapy for hemophilia. J. Gene Med. 3, 3-20.
DEMAISON, C., PARSLEY, K., BROUNS, G., SCHERR, M., BATTMER, K., KINNON, C., GREZ, M., THRASHER, A.J. (2002). High-level transduction and gene expression in hematopoietic repopulating cells using a human immunodeficiency virus type 1-based lentiviral vector containing an internal spleen focus forming virus promoter. Hum. Gene Ther. 13, 803-813.
DO, H., HEALEY, J.F., WALLER, E.K., LOLLAR, P. (1999). Expression of factor VIII by murine liver sinusoidal endothelial cells. J. Biol. Chem. 274, 19587-19592.
DWARKI, V.J., BELLONI, P., NIJJAR, T., SMITH, J., COUTO, L., RABIER, M., CLIFT, S., BERNS, A., COHEN, L.K. (1995). Gene therapy for hemophilia A: production of therapeutic levels of human factor VIII in vivo in mice. Proc. Natl. Acad. Sci. U.S.A. 92, 1023-1027.
EVANS, G.L., MORGAN, R.A. (1998). Genetic induction of immune tolerance to human clotting factor VIII in a mouse model for hemophilia A. Proc. Natl. Acad. Sci. U.S.A. 95, 5734-5739.
FAKHARZADEH, S.S., ZHANG, Y., SARKAR, R., KAZAZIAN, H.H. JR. (2000). Correction of the coagulation defect in hemophilia A mice through factor VIII expression in skin. Blood 95, 2799-2805.
FOLLENZI, A., AILLES, L.E., BAKOVIC, S., GEUNA, M., NALDINI, L. (2000). Gene transfer by lentiviral vectors is limited by nuclear translocation and rescued by HIV-1 pol sequences. Nat. Genet. 25, 217-222.
FOLLENZI, A., NALDINI, L. (2002). Generation of HIV-1 derived lentiviral vectors. Methods Enzymol. 346, 454-465.
GARCIA-MARTIN, C., CHUAH, M.K.L., VAN DAMME, A., ROBINSON, K.E., VANZIELEGHEM, B., SAINT-REMY, J.M., GALLARDO, D., OFOSU, F.A., VANDENDRIESSCHE, T., HORTELANO, G. (2002). Therapeutic levels of human factor VIII in mice implanted with encapsulated cells: potential for gene therapy of hemophilia A. J. Gene Med. 4, 215-223.
GEHLING, U.M., ERGÜN, S., SCHUMACHER, U., WAGENER, C., PANTEL, K., OTTE, M., SCHUCH, G., SCHAFHAUSEN, P., MENDE, T., KILIC, N., KLUGE, K., SCHÄFER, B., HOSSFELD, D.K., FIEDLER, W. (2000). In vitro differentiation of endothelial cells from AC133-positive progenitor cells. Blood 95, 3106-3112.
GRANT, M.B., MAY, W.S., CABALLERO, S., BROWN, G.A.J., GUTHRIE, S.M., MAMES, R.N., BYRNE, B.J., VAUGHT, T., SPOERRI, P.E., PECK, A.B., SCOTT, E.W. (2002). Adult hematopoietic stem cells provide functional hemangioblast activity during retinal neovascularization. Nat. Med. 6, 607-612.
GREENGARD, J.S., JOLLY, D.J. (1999). Animal testing of retroviral-mediated gene therapy for factor FVIII deficiency. Thromb. Haemost. 82, 555-561.
HOEBEN, R.C., VAN DER JAGT, R.C., SCHOUTE, F., VAN TILBURG, N.H., VERBEET, M.P., BRIET, E., VAN ORMONDT, H., VAN DER EB, A.J. (1990). Expression of functional factor VIII in primary human skin fibroblasts after retrovirus-mediated gene transfer. J. Biol. Chem. 265, 7318-7323.
HOEBEN, R.C., EINERHAND, M.P., BRIET, E., VAN ORMONDT, H., VALERIO, D., VAN DER EB, A.J. (1992). Toward gene therapy in haemophilia A: retrovirus-mediated transfer of a factor VIII gene into murine hematopoietic progenitor cells. Thromb. Haemost. 67, 341-345.
HOLLESTELLE, M.J., THINNES, T., CRAIN, K., STIKO, A., KRUIJT, J.K., VAN BERKEL, T.J.C., LOSKUTOFF, D.J., VAN MOURIK, J.A. (2001). Tissue distribution of FVIII gene expression in vivo - a closer look. Thromb. Haemost. 86, 855-861.
HOOTS, W.K. (2001). History of plasma-product safety. Transfus. Med. Rev. 15 (2 suppl. 1), 3-10.
IWAGURO, H., YAMAGUCHI, J., KALKA, C., MURASAWA, S., MASUDA, H., HAYASHI, S., SILVER, M., LI, T., ISNER, J.M., ASAHARA, T. (2002). Endothelial progenitor cell VEGF gene transfer for vascular regeneration. Circulation 105, 732-738.
JACKSON, K.A., MAJKA, S.M., WANG, H., POCIUS, J., HARTLEY, C.J., MAJEWSKY, M.W., ENTMAN, M.L., MICHAEL, L.H., HIRSCHI, K.K., GOODELL, M.A. (2001). Regeneration of ischemic cardiac muscle and vascular endothelium by adult stem cells. J. Clin. Invest. 107, 1395-1402.
KAUFMAN, R.J. (1999). Advances toward gene therapy for hemophilia at the millennium. Hum. Gene. Ther. 10, 2091-2107.
LIN, Y., WEISDORF, D.J., SOLOVEY, A., HEBBEL, R.P. (2000). Origins of circulating endothelial cells and endothelial outgrowth from blood. J. Clin. Invest. 105, 71-77.
LIN, Y., CHANG, L., SOLOVEY, A., HEALEY, J.F., LOLLAR, P., HEBBEL, R.P. (2002). Use of blood outgrowth endothelial cells for gene therapy for hemophilia A. Blood 99, 457-462.
LOZIER, J.N., METZGER, M.E., DONAHUE, R.E., MORGAN, R.A. (1999). Adenovirus-mediated expression of human coagulation factor IX in the rhesus macaque is associated with dose-limiting toxicity. Blood 94, 3968-3975.
MANNUCCI, P.M., GIANGRANDE, P.L.F. (2000). Choice of replacement therapy for hemophilia: recombinant products only? Hematol. J. 1, 72-76.
MANNUCCI, P.M., TUDDENHAM, E.G.D. (2001). The hemophilias - from royal genes to gene therapy. N. Engl. J. Med. 344, 1773-1779.
MUROHARA, T. (2001). Therapeutic vasculogenesis using human cord blood-derived endothelial progenitors. Trends Cardiovasc. Med. 11, 303-307.
MURRAY, P.D.F. (1932). The development in vitro of blood of the early chick embryo. Proc. Roy. Soc. 111, 497-521.
MYERS, G., JOSEPHS, S.F., BERZOFSKY, J.A., RABSON, A.B., SMITH, T.F., WONG-STAAL, F. (eds.) (1989). Human retroviruses and AIDS 1989- a compilation and analysis of nucleic acid and amino acid sequences. Los Alamos National Laboratory, Los Alamos, NM.
QUIRICI, N., SOLIGO, D., CANEVA, L., SERVIDA, F., BOSSOLASCO, P., LAMBERTENGHI DELILIERS, G. (2001). Differentiation and expansion of endothelial cells from human bone marrow CD133+ cells. Br. J. Haematol. 115, 186-194.
REDING, M.T., WU, H., KRAMPF, M., OKITA, D.K., DIETHELM-OKITA, B.M., KEY, N.S., CONTI-FINE, B.M. (1999). CD4+ T cell response to FVIII in hemophilia A, acquired hemophilia, and healthy subjects. Thromb. Haemost. 82, 509-515.
ROSENBERG, J.B., GREENGARD, J.S., MONTGOMERY, R.R. (2000). Genetic induction of a releasable pool of factor VIII in human endothelial cells. Arterioscler. Thromb. Vasc. Biol. 20, 2689-2695.
ROTH, D.A., TAWA, N.E. JR., O'BRIEN, J.M., TRECO, D.A., SELDEN, R.F. (2001). Nonviral transfer of the gene encoding coagulation factor VIII in patients with severe hemophilia. N. Engl. J. Med. 344, 1735-1742.
SABIN, F. (1920). Studies on the origin of blood vessels and of red blood corpuscules as seen in the living blastoderm of chicks during the second day of incubation. Carnegie Inst. Pub. 9-36, 214-262.
SIRVEN, A., PFLUMIO, F., ZENNOU, V., TITEUX, M., VAINCHENKER, W., COULOMBEL, L., DUBART-KUPPERSCHMITT, A., CHARNEAU, P. (2000). The human immunodeficiency virus type-1 central DNA flap is a crucial determinant for lentiviral vector nuclear import and gene transduction of hematopoietic stem cells. Blood 96, 4103-4110.
TAGLIAVACCA, L., WANG, Q., KAUFMAN, R.J. (2000). ATP-dependent dissociation of non-disulfide-linked aggregates of coagulation factor VIII is a rate-limiting step for secretion. Biochemistry 39, 1973-1981.
TEITEL, J.M. (2000). Viral safety of haemophila treatment products. Ann. Med. 32, 485-492.
TONN, T., HERDER, C., BECKER, S., SEIFRIED, E., GREZ, M. (2002). Generation and characterization of human hematopoietic cell lines expressing factor VIII. J. Hematoth. Stem Cell Res. 11, 695-704.
VANDENDRIESSCHE, T., COLLEN, D., CHUAH, M.K.L. (2001). Viral vector-mediated gene therapy for hemophilia. Curr. Gene Ther. 1, 301-315.
WHITE II, G.C., GREENWOOD, R., ESCOBAR, M., FRELINGER, J.A. (2000). Hemophilia factor VIII therapy. Immunological tolerance. A clinical perspective. Haematologica 85 (10 suppl.), 113-116.
WHITE II, G.C. (2001). Gene therapy in hemophilia: clinical trials update. Thromb. Haemost. 86, 172-177.
WION, K.L., KELLY, D., SUMMERFIELD, J.A., TUDDENHAM, E.G., LAWN, R.M. (1985). Distribution of FVIII mRNA and antigen in human liver and other tissues . Nature 317, 726-729.
YANG, Y., JOOSS, K.U., SU, Q., ERTL, H.C., WILSON, J.M. (1996). Immune responses to viral antigens versus transgene product in the elimination of recombinant adenovirus-infected hepatocytes in vivo. Gene Ther. 3, 137-144.
ZELECHOWSKA, M.G., VAN MOURIK, J.A., BRODNIEWICZ-PROBA, T. (1985). Ultrastructural localization of factor VIII procoagulant antigen in human liver hepatocytes. Nature 317, 729-730.
ZENNOU, V., PETIT, C., GUETARD, D., NEHRBASS, U., MONTAGNIER, L., CHARNEAU, P. (2000). HIV-1 genome nuclear import is mediated by a central DNA flap. Cell 101, 173-185.
ZUFFEREY, R., NAGY, D., MANDEL, R.J., NALDINI, L., TRONO, D. (1997). Multiply attenuated lentiviral vector achieves efficient gene delivery in vivo. Nature Biotechnol. 15, 871-875.

## Claims

1. A method for the preparation of human endothelial cells expressing a blood coagulation factor, comprising
a) obtaining endothelial precursor cells from a human cord blood sample:
b) contacting in vitro said human cord blood-derived endothelial precursor cells with at least one growth factor, wherein the at least one growth factor promotes the differentiation of endothelial precursor cells into mature endothelial cells; and
c) transducing the mature endothelial cells with DNA encoding the blood coagulation factor, or transducing, prior to step b), the endothelial precursor cells with DNA encoding the blood coagulation factor.

2. A method according to claim 1, wherein the human cord blood-derived endothelial precursor cells express at least one cell surface marker selected from the group consisting of CD34, AC133, CD146 and FGF1-R.

3. A method according to claim 1 or 2 wherein step a) comprises enriching CD34-positive cells, AC133-positive cells, CD146-positive cells and/or FGF1-R-positive cells from cord blood.

4. A method according to any one of claims 1 to 3 wherein a retroviral vector, preferably a lentiviral vector is used for transducing the cells.

5. A method according to claim 4, wherein the blood coagulation factor is human factor VIII.

6. A method for the production of a blood coagulation factor, comprising
a) obtaining a population of human endothelial cells by a method according to any one of claims 1-5; and optionally generating an immortalized cell line from said population of human endothelial cells;
b) culturing saidpopulation of human endothelial cells or saidcell line under suitable conditions; and
c) isolating the blood coagulation factor from the cell culture medium.

7. A method according to claim 6 wherein the blood coagulation factor is human blood coagulation factor VIII or IX.

8. A method according to claim 6 or 7 wherein the recombinant DNA encoding a blood coagulation factor encodes a mutein of human blood coagulation factor VIII.

9. A method according to claim 8 wherein the mutein of human factor VIII at least partially lacks the B domain of wild type factor VIII.

10. A method according to any one of claims 6 to 9, wherein the recombinant DNA encoding a blood coagulation factor is a modified factor VIII cDNA, wherein
(i) at least part of the B-domain of the wild type factor VIII cDNA has been deleted,
(ii) at least one intron has been inserted into at least one location of the factor VIII cDNA, and/or
(iii) at least one nucleotide of the wild type cDNA sequence of human factor VIII is substituted.

11. A method according to any one of claims 6 to 10, wherein at least 75% of the cells express at least one marker selected from the group consisting of CD144, CD31, CD146 and LDL-receptor.

12. A method according to any one of claims 6 to 11, wherein at least 75% of the cells endogenously express VWF.

13. A method according to any one of claims 6 to 12, wherein the blood coagulation factor is blood coagulation factor VIII and the isolation step includes purifying blood coagulation factor VIII in the presence of von Willebrand factor.

14. A method according to any one of claims 6 to 13, wherein the blood coagulation factor is subjected to virus inactivation treatment.

15. The use of a population of human endothelial cells for the preparation of a medicament for the treatment of hemophilia A or hemophilia B, wherein said use comprises obtaining said population of human endothelial cells by a method according to any one of claims 1-5.

16. The use according to claim 15, wherein cells secreting human blood coagulation factor VIII are administered to an individual suffering from hemophilia A.

## Patentansprüche

1. Verfahren zur Herstellung menschlicher Endothelzellen, die einen Blutgerinnungsfaktor exprimieren, umfassend
a) das Erhalten von Endothel-Präkursorzellen aus einer menschlichen Nabelschnurblutprobe,
b) das In-vitro-Kontaktieren der aus menschlichem Nabelschnurblut stammenden Endothel-Präkursorzellen mit mindestens einem Wachstumsfaktor, wobei der mindestens eine Wachstumsfaktor die Differenzierung der Endothel-Präkursorzellen zu reifen Endothelzellen fördert; und
c) das Transduzieren der reifen Endothelzellen mit DNA, die den Blutgerinnungsfaktor kodiert, oder vor Schritt b) das Transduzieren der Endothel-Präkursorzellen mit DNA, die den Blutgerinnungsfaktor kodiert.

2. Verfahren nach Anspruch 1, wobei die aus menschlichem Nabelschnurblut stammenden Endothel-Präkursorzellen mindestens einen Zelloberflächenmarker, ausgewählt aus der Gruppe, bestehend aus CD34, AC133, CD 146 und FGF1-R, exprimieren.

3. Verfahren nach Anspruch 1 oder 2, wobei Schritt a) das Anreichern CD34-positiver Zellen, AC133-positiver Zellen, CD146-positiver Zellen und/oder FGF1-R-positiver Zellen aus Nabelschnurblut umfasst.

4. Verfahren nach einem der Ansprüche 1 bis 3, wobei ein Retrovirusvektor, bevorzugt ein Lentivirusvektor, für die Transduktion der Zellen verwendet wird.

5. Verfahren nach Anspruch 4, wobei der Blutgerinnungsfaktor der menschliche Faktor VIII ist.

6. Verfahren zur Herstellung eines Blutgerinnungsfaktors, umfassend
a) das Erhalten einer Population menschlicher Endothelzellen durch ein Verfahren nach einem der Ansprüche 1 bis 5; und gegebenenfalls das Erzeugen einer immortalisierten Zelllinie aus der Population menschlicher Endothelzellen;
b) das Kultivieren der Population menschlicher Endothelzellen oder der Zelllinie unter geeigneten Bedingungen und
c) das Isolieren des Blutgerinnungsfaktors aus dem Zellkulturmedium.

7. Verfahren nach Anspruch 6, wobei der Blutgerinnungsfaktor der menschliche Blutgerinnungsfaktor VIII oder IX ist.

8. Verfahren nach Anspruch 6 oder 7, wobei die rekombinante DNA, die einen Blutgerinnungsfaktor kodiert, ein Mutein des menschlichen Blutgerinnungsfaktors VIII kodiert.

9. Verfahren nach Anspruch 8, wobei es dem Mutein des menschlichen Faktors VIII zumindest teilweise an der B-Domäne des Wildtyp-Faktors VIII mangelt.

10. Verfahren nach einem der Ansprüche 6 bis 9, wobei die rekombinante DNA, die einen Blutgerinnungsfaktor kodiert, eine modifizierte Faktor VIII-cDNA ist, in der
(i) zumindest ein Teil der B-Domäne der Wildtyp-Faktor VIII-cDNA deletiert worden ist,
(ii) mindestens ein Intron in mindestens eine Stelle der Faktor VIII-cDNA eingeführt worden ist und/oder
(iii) mindestens ein Nucleotid der Wildtyp-cDNA-Sequenz des menschlichen Faktors VIII substituiert ist.

11. Verfahren nach einem der Ansprüche 6 bis 10, wobei mindestens 75 % der Zellen mindestens einen Marker, ausgewählt aus der Gruppe, bestehend aus CD 144, CD31, CD 146 und LDL-Rezeptor, exprimieren.

12. Verfahren nach einem der Ansprüche 6 bis 11, wobei mindestens 75 % der Zellen VWF endogen exprimieren.

13. Verfahren nach einem der Ansprüche 6 bis 12, wobei der Blutgerinnungsfaktor der Blutgerinnungsfaktor VIII ist und der Isolierschritt das Reinigen des Blutgerinnungsfaktors VIII in Gegenwart des von Willebrand-Faktors umfasst.

14. Verfahren nach einem der Ansprüche 6 bis 13, wobei der Blutgerinnungsfaktor einer Virusinaktivierungsbehandlung unterzogen wird.

15. Verwendung einer Population menschlicher Endothelzellen zur Herstellung eines Medikaments zur Behandlung von Hämophilie A oder Hämophilie B, wobei die Verwendung das Erhalten einer Population menschlicher Endothelzellen durch ein Verfahren nach einem der Ansprüche 1 bis 5 umfasst.

16. Verwendung nach Anspruch 15, wobei Zellen, die den menschlichen Blutgerinnungsfaktor VIII sekretieren, einem Individuum, das an Hämophilie A leidet, verabreicht werden.

## Revendications

1. Procédé de préparation de cellules endothéliales humaines exprimant un facteur de coagulation sanguine, comprenant les étapes consistant à :
a) obtenir des cellules précurseurs endothéliales à partir d'un échantillon de sang du cordon
b) mettre en contact *in vitro* lesdites cellules précurseurs endothéliales dérivées du sang du cordon humain avec au moins un facteur de croissance, dans lequel l'au moins un facteur de croissance favorise la différenciation des cellules précurseurs endothéliales en cellules endothéliales matures ; et
c) transduire les cellules endothéliales matures avec de l'ADN codant pour le facteur de coagulation sanguine, ou transduire, avant l'étape b), les cellules précurseurs endothéliales avec de l'ADN codant pour le facteur de coagulation sanguine.

2. Procédé selon la revendication 1, dans lequel les cellules précurseurs endothéliales dérivées du sang du cordon humain expriment au moins un marqueur de surface cellulaire choisi dans le groupe comprenant le CD34, le AC133, le CD146 et le FGF1-R.

3. Procédé selon la revendication 1 ou 2 dans lequel l'étape a) comprend l'étape consistant à enrichir les cellules CD34 +, les cellules AC133 +, les cellules CD146 + et/ou les cellules FGF1-R + à partir du sang du cordon.

4. Procédé selon l'une quelconque des revendications 1 à 3 dans lequel un vecteur rétroviral, de préférence un vecteur lentiviral, est utilisé pour transduire les cellules.

5. Procédé selon la revendication 4, dans lequel le facteur de coagulation sanguine est le facteur VIII humain.

6. Procédé de production d'un facteur de coagulation sanguine, comprenant les étapes consistant à :
a) obtenir une population de cellules endothéliales humaines par un procédé selon l'une quelconque des revendications 1 à 5 ; et générer éventuellement une lignée cellulaire immortalisée à partir de ladite population de cellules endothéliales humaines ;
b) cultiver ladite population de cellules endothéliales humaines ou ladite lignée cellulaire dans des conditions appropriées ; et
c) isoler le facteur de coagulation sanguine à partir du milieu de culture cellulaire.

7. Procédé selon la revendication 6 dans lequel le facteur de coagulation sanguine est le facteur de coagulation sanguine humain VIII ou IX.

8. Procédé selon la revendication 6 ou 7 dans lequel l'ADN recombinant codant pour un facteur de coagulation sanguine code pour une mutéine du facteur de coagulation sanguine humain VIII.

9. Procédé selon la revendication 8 dans lequel la mutéine du facteur VIII humain est dépourvue au moins partiellement du domaine B du facteur VIII de type sauvage.

10. Procédé selon l'une quelconque des revendications 6 à 9, dans lequel l'ADN recombinant codant pour un facteur de coagulation sanguine est un ADNc du facteur VIII modifié, dans lequel
(i) au moins une partie du domaine B de l'ADNc du facteur VIII de type sauvage a été enlevée,
(ii) au moins un intron a été inséré à l'intérieur d'au moins une position de l'ADNc du facteur VIII, et/ou
(iii) au moins un nucléotide de la séquence d'ADNc de type sauvage du facteur VIII humain est substitué.

11. Procédé selon l'une quelconque des revendications 6 à 10, dans lequel au moins 75 % des cellules expriment au moins un marqueur choisi dans le groupe comprenant le CD144, le CD31, le CD146 et un récepteur du LDL.

12. Procédé selon l'une quelconque des revendications 6 à 11, dans lequel au moins 75 % des cellules expriment le VWF de manière endogène.

13. Procédé selon l'une quelconque des revendications 6 à 12, dans lequel le facteur de coagulation sanguine est le facteur de coagulation sanguine VIII et l'étape d'isolement comprend la purification du facteur de coagulation sanguine VIII en présence du facteur de von Willebrand.

14. Procédé selon l'une quelconque des revendications 6 à 13, dans lequel le facteur de coagulation
sanguine est soumis à un traitement d'inactivation des virus.

15. Utilisation d'une population de cellules endothéliales humaines dans la préparation d'un médicament destiné à traiter l'hémophilie A ou l'hémophilie B, ladite utilisation comprenant l'obtention de ladite population de cellules endothéliales humaines par un procédé selon l'une quelconque des revendications 1 à 5.

16. Utilisation selon la revendication 15, dans laquelle les cellules sécrétant le facteur de coagulation sanguine humain VIII sont administrées à un individu souffrant d'hémophilie A.


### SEQUENCE LISTING

<110> DRK-Blutspendedienst Baden-Wurttemberg - Hessen gGmbH
   <120> Expression of proteins in cord blood-derived endothelial cells <130> CBECs
   <160> 3
   <170> PatentIn version 3.1
<210> 1
   <211> 25
   <212> DNA
   <213> Artificial sequence
   <220>
   <223> Primer 1
   <400> 1
   ccatcgatac aaatggcatt catcc 25
<210> 2
   <211> 36
   <212> DNA
   <213> Artificial Sequence
   <220>
   <223> Primer 2
   <400> 2
   ccatcgatct cgagccaaag tggatctctg ctgtcc 36
<210> 3
   <211> 2351
   <212> PRT
   <213> homo sapiens
   <400> 3
   Met Gln Ile Glu Leu Ser Thr Cys Phe Phe Leu Cys Leu Leu Arg Phe
   1               5                   10                  15
   Cys Phe Ser Ala Thr Arg Arg Tyr Tyr Leu Gly Ala Val Glu Leu Ser
               20                  25                  30
   Trp Asp Tyr Met Gln Ser Asp Leu Gly Glu Leu Pro Val Asp Ala Arg
           35                  40                  45
   Phe Pro Pro Arg Val Pro Lys Ser Phe Pro Phe Asn Thr Ser Val Val
       50                  55                  60
   Tyr Lys Lys Thr Leu Phe Val Glu Phe Thr Asp His Leu Phe Asn Ile
   65                  70                  75                  80
   Ala Lys Pro Arg Pro Pro Trp Met Gly Leu Leu Gly Pro Thr Ile Gln
                   85                  90                  95
   Ala Glu Val Tyr Asp Thr Val Val Ile Thr Leu Lys Asn Met Ala Ser
               100                 105                 110
   His Pro Val Ser Leu His Ala Val Gly Val Ser Tyr Trp Lys Ala Ser
           115                 120                 125
   Glu Gly Ala Glu Tyr Asp Asp Gln Thr Ser Gln Arg Glu Lys Glu Asp
       130                 135                 140
   Asp Lys Val Phe Pro Gly Gly Ser His Thr Tyr Val Trp Gln Val Leu
   145                 150                 155                 160
   Lys Glu Asn Gly Pro Met Ala Ser Asp Pro Leu Cys Leu Thr Tyr Ser
                   165                 170                 175
   Tyr Leu Ser His Val Asp Leu Val Lys Asp Leu Asn Ser Gly Leu Ile
               180                 185                 190
   Gly Ala Leu Leu Val Cys Arg Glu Gly Ser Leu Ala Lys Glu Lys Thr
           195                 200                 205
   Gln Thr Leu His Lys Phe Ile Leu Leu Phe Ala Val Phe Asp Glu Gly
       210                 215                 220
   Lys Ser Trp His Ser Glu Thr Lys Asn Ser Leu Met Gln Asp Arg Asp
   225                 230                 235                 240
   Ala Ala Ser Ala Arg Ala Trp Pro Lys Met His Thr Val Asn Gly Tyr
                   245                 250                 255
   Val Asn Arg Ser Leu Pro Gly Leu Ile Gly Cys His Arg Lys Ser Val
               260                 265                 270
   Tyr Trp His Val Ile Gly Met Gly Thr Thr Pro Glu Val His Ser Ile
           275                 280                 285
   Phe Leu Glu Gly His Thr Phe Leu Val Arg Asn His Arg Gln Ala Ser
       290                 295                 300
   Leu Glu Ile Ser Pro Ile Thr Phe Leu Thr Ala Gln Thr Leu Leu Met
   305                 310                 315                 320
   Asp Leu Gly Gln Phe Leu Leu Phe Cys His Ile Ser Ser His Gln His
                   325                 330                 335
   Asp Gly Met Glu Ala Tyr Val Lys Val Asp Ser Cys Pro Glu Glu Pro
               340                 345                 350
   Gln Leu Arg Met Lys Asn Asn Glu Glu Ala Glu Asp Tyr Asp Asp Asp
           355                 360                 365
   Leu Thr Asp Ser Glu Met Asp Val Val Arg Phe Asp Asp Asp Asn Ser
       370                 375                 380
   Pro Ser Phe Ile Gln Ile Arg Ser Val Ala Lys Lys His Pro Lys Thr
   385                 390                 395                 400
   Trp Val His Tyr Ile Ala Ala Glu Glu Glu Asp Trp Asp Tyr Ala Pro
                   405                 410                 415
   Leu Val Leu Ala Pro Asp Asp Arg Ser Tyr Lys Ser Gln Tyr Leu Asn
               420                 425                 430
   Asn Gly Pro Gln Arg Ile Gly Arg Lys Tyr Lys Lys Val Arg Phe Met
           435                 440                 445
   Ala Tyr Thr Asp Glu Thr Phe Lys Thr Arg Glu Ala Ile Gln His Glu
       450                 455                 460
   Ser Gly Ile Leu Gly Pro Leu Leu Tyr Gly Glu Val Gly Asp Thr Leu
   465                 470                 475                 480
   Leu Ile Ile Phe Lys Asn Gln Ala Ser Arg Pro Tyr Asn Ile Tyr Pro
                   485                 490                 495
   His Gly Ile Thr Asp Val Arg Pro Leu Tyr Ser Arg Arg Leu Pro Lys
               500                 505                 510
   Gly Val Lys His Leu Lys Asp Phe Pro Ile Leu Pro Gly Glu Ile Phe
           515                 520                 525
   Lys Tyr Lys Trp Thr Val Thr Val Glu Asp Gly Pro Thr Lys Ser Asp
       530                 535                 540
   Pro Arg Cys Leu Thr Arg Tyr Tyr Ser Ser Phe Val Asn Met Glu Arg
   545                 550                 555                 560
   Asp Leu Ala Ser Gly Leu Ile Gly Pro Leu Leu Ile Cys Tyr Lys Glu
                   565                 570                 575
   Ser Val Asp Gln Arg Gly Asn Gln Ile Met Ser Asp Lys Arg Asn Val
               580                 585                 590
   Ile Leu Phe Ser Val Phe Asp Glu Asn Arg Ser Trp Tyr Leu Thr Glu
           595                 600                 605
   Asn Ile Gln Arg Phe Leu Pro Asn Pro Ala Gly Val Gln Leu Glu Asp
       610                 615                 620
   Pro Glu Phe Gln Ala Ser Asn Ile Met His Ser Ile Asn Gly Tyr Val
   625                 630                 635                 640
   Phe Asp Ser Leu Gln Leu Ser Val Cys Leu His Glu Val Ala Tyr Trp
                   645                 650                 655
   Tyr Ile Leu Ser Ile Gly Ala Gln Thr Asp Phe Leu Ser Val Phe Phe
               660                 665                 670
   Ser Gly Tyr Thr Phe Lys His Lys Met Val Tyr Glu Asp Thr Leu Thr
           675                 680                 685
   Leu Phe Pro Phe Ser Gly Glu Thr Val Phe Met Ser Met Glu Asn Pro
       690                 695                 700
   Gly Leu Trp Ile Leu Gly Cys His Asn Ser Asp Phe Arg Asn Arg Gly
   705                 710                 715                 720
   Met Thr Ala Leu Leu Lys Val Ser Ser Cys Asp Lys Asn Thr Gly Asp
                   725                 730                 735
   Tyr Tyr Glu Asp Ser Tyr Glu Asp Ile Ser Ala Tyr Leu Leu Ser Lys
               740                 745                 750
   Asn Asn Ala Ile Glu Pro Arg Ser Phe Ser Gln Asn Ser Arg His Pro
           755                 760                 765
   Ser Thr Arg Gln Lys Gln Phe Asn Ala Thr Thr Ile Pro Glu Asn Asp
       770                 775                 780
   Ile Glu Lys Thr Asp Pro Trp Phe Ala His Arg Thr Pro Met Pro Lys
   785                 790                 795                 800
   Ile Gln Asn Val Ser Ser Ser Asp Leu Leu Met Leu Leu Arg Gln Ser
   805 810 815
   Pro Thr Pro His Gly Leu Ser Leu Ser Asp Leu Gln Glu Ala Lys Tyr
   820 825 830
   Glu Thr Phe Ser Asp Asp Pro Ser Pro Gly Ala Ile Asp Ser Asn Asn
   835 840 845
   Ser Leu Ser Glu Met Thr His Phe Arg Pro Gln Leu His His Ser Gly
   850 855 860
   Asp Met Val Phe Thr Pro Glu Ser Gly Leu Gln Leu Arg Leu Asn Glu
   865 870 875 880
   Lys Leu Gly Thr Thr Ala Ala Thr Glu Leu Lys Lys Leu Asp Phe Lys
   885 890 895
   Val Ser Ser Thr Ser Asn Asn Leu Ile Ser Thr Ile Pro Ser Asp Asn
   900 905 910
   Leu Ala Ala Gly Thr Asp Asn Thr Ser Ser Leu Gly Pro Pro Ser Met
   915 920 925
   Pro Val His Tyr Asp Ser Gln Leu Asp Thr Thr Leu Phe Gly Lys Lys
   930 935 940
   Ser Ser Pro Leu Thr Glu Ser Gly Gly Pro Leu Ser Leu Ser Glu Glu
   945 950 955 960
   Asn Asn Asp Ser Lys Leu Leu Glu Ser Gly Leu Met Asn Ser Gln Glu
   965 970 975
   Ser Ser Trp Gly Lys Asn Val Ser Ser Thr Glu Ser Gly Arg Leu Phe
   980 985 990
   Lys Gly Lys Arg Ala His Gly Pro Ala Leu Leu Thr Lys Asp Asn Ala
   995 1000 1005
   Leu Phe Lys Val Ser Ile Ser Leu Leu Lys Thr Asn Lys Thr Ser
   1010 1015 1020
   Asn Asn Ser Ala Thr Asn Arg Lys Thr His Ile Asp Gly Pro Ser
   1025 1030 1035
   Leu Leu Ile Glu Asn Ser Pro Ser Val Trp Gln Asn Ile Leu Glu
   1040 1045 1050
   Ser Asp Thr Glu Phe Lys Lys Val Thr Pro Leu Ile His Asp Arg
   1055 1060 1065
   Met Leu Met Asp Lys Asn Ala Thr Ala Leu Arg Leu Asn His Met
   1070 1075 1080
   Ser Asn Lys Thr Thr Ser Ser Lys Asn Met Glu Met Val Gln Gln
   1085 1090 1095
   Lys Lys Glu Gly Pro Ile Pro Pro Asp Ala Gln Asn Pro Asp Met
   1100 1105 1110
   Ser Phe Phe Lys Met Leu Phe Leu Pro Glu Ser Ala Arg Trp Ile
   1115 1120 1125
   Gln Arg Thr His Gly Lys Asn Ser Leu Asn Ser Gly Gln Gly Pro
   1130 1135 1140
   Ser Pro Lys Gln Leu Val Ser Leu Gly Pro Glu Lys Ser Val Glu
   1145 1150 1155
   Gly Gln Asn Phe Leu Ser Glu Lys Asn Lys Val Val Val Gly Lys
   1160 1165 1170
   Gly Glu Phe Thr Lys Asp Val Gly Leu Lys Glu Met Val Phe Pro
   1175 1180 1185
   Ser Ser Arg Asn Leu Phe Leu Thr Asn Leu Asp Asn Leu His Glu
   1190 1195 1200
   Asn Asn Thr His Asn Gln Glu Lys Lys Ile Gln Glu Glu Ile Glu
   1205 1210 1215
   Lys Lys Glu Thr Leu Ile Gln Glu Asn Val Val Leu Pro Gln Ile
   1220 1225 1230
   His Thr Val Thr Gly Thr Lys Asn Phe Met Lys Asn Leu Phe Leu
   1235 1240 1245
   Leu Ser Thr Arg Gln Asn Val Glu Gly Ser Tyr Asp Gly Ala Tyr
   1250 1255 1260
   Ala Pro Val Leu Gln Asp Phe Arg Ser Leu Asn Asp Ser Thr Asn
   1265 1270 1275
   Arg Thr Lys Lys His Thr Ala His Phe Ser Lys Lys Gly Glu Glu
   1280 1285 1290
   Glu Asn Leu Glu Gly Leu Gly Asn Gln Thr Lys Gln Ile Val Glu
   1295 1300 1305
   Lys Tyr Ala Cys Thr Thr Arg Ile Ser Pro Asn Thr Ser Gln Gln
   1310 1315 1320
   Asn Phe Val Thr Gln Arg Ser Lys Arg Ala Leu Lys Gln Phe Arg
   1325 1330 1335
   Leu Pro Leu Glu Glu Thr Glu Leu Glu Lys Arg Ile Ile Val Asp
   1340 1345 1350
   Asp Thr Ser Thr Gln Trp Ser Lys Asn Met Lys His Leu Thr Pro
   1355 1360 1365
   Ser Thr Leu Thr Gln Ile Asp Tyr Asn Glu Lys Glu Lys Gly Ala
   1370 1375 1380
   Ile Thr Gln Ser Pro Leu Ser Asp Cys Leu Thr Arg Ser His Ser
   1385 1390 1395
   Ile Pro Gln Ala Asn Arg Ser Pro Leu Pro Ile Ala Lys Val Ser
   1400 1405 1410
   Ser Phe Pro Ser Ile Arg Pro Ile Tyr Leu Thr Arg Val Leu Phe
   1415 1420 1425
   Gln Asp Asn Ser Ser His Leu Pro Ala Ala Ser Tyr Arg Lys Lys
   1430 1435 1440
   Asp Ser Gly Val Gln Glu Ser Ser His Phe Leu Gln Gly Ala Lys
   1445 1450 1455
   Lys Asn Asn Leu Ser Leu Ala Ile Leu Thr Leu Glu Met Thr Gly
   1460 1465 1470
   Asp Gln Arg Glu Val Gly Ser Leu Gly Thr Ser Ala Thr Asn Ser
   1475 1480 1485
   Val Thr Tyr Lys Lys Val Glu Asn Thr Val Leu Pro Lys Pro Asp
   1490 1495 1500
   Leu Pro Lys Thr Ser Gly Lys Val Glu Leu Leu Pro Lys Val His
   1505 1510 1515
   Ile Tyr Gln Lys Asp Leu Phe Pro Thr Glu Thr Ser Asn Gly Ser
   1520 1525 1530
   Pro Gly His Leu Asp Leu Val Glu Gly Ser Leu Leu Gln Gly Thr
   1535 1540 1545
   Glu Gly Ala Ile Lys Trp Asn Glu Ala Asn Arg Pro Gly Lys Val
   1550 1555 1560
   Pro Phe Leu Arg Val Ala Thr Glu Ser Ser Ala Lys Thr Pro Ser
   1565 1570 1575
   Lys Leu Leu Asp Pro Leu Ala Trp Asp Asn His Tyr Gly Thr Gln
   1580 1585 1590
   Ile Pro Lys Glu Glu Trp Lys Ser Gln Glu Lys Ser Pro Glu Lys
   1595 1600 1605
   Thr Ala Phe Lys Lys Lys Asp Thr Ile Leu Ser Leu Asn Ala Cys
   1610 1615 1620
   Glu Ser Asn His Ala Ile Ala Ala Ile Asn Glu Gly Gln Asn Lys
   1625 1630 1635
   Pro Glu Ile Glu Val Thr Trp Ala Lys Gln Gly Arg Thr Glu Arg
   1640 1645 1650
   Leu Cys Ser Gln Asn Pro Pro Val Leu Lys Arg His Gln Arg Glu
   1655 1660 1665
   Ile Thr Arg Thr Thr Leu Gln Ser Asp Gln Glu Glu Ile Asp Tyr
   1670 1675 1680
   Asp Asp Thr Ile Ser Val Glu Met Lys Lys Glu Asp Phe Asp Ile
   1685 1690 1695
   Tyr Asp Glu Asp Glu Asn Gln Ser Pro Arg Ser Phe Gln Lys Lys
   1700 1705 1710
   Thr Arg His Tyr Phe Ile Ala Ala Val Glu Arg Leu Trp Asp Tyr
   1715 1720 1725
   Gly Met Ser Ser Ser Pro His Val Leu Arg Asn Arg Ala Gln Ser
   1730 1735 1740
   Gly Ser Val Pro Gln Phe Lys Lys Val Val Phe Gln Glu Phe Thr
   1745 1750 1755
   Asp Gly Ser Phe Thr Gln Pro Leu Tyr Arg Gly Glu Leu Asn Glu
   1760 1765 1770
   His Leu Gly Leu Leu Gly Pro Tyr Ile Arg Ala Glu Val Glu Asp
   1775 1780 1785
   Asn Ile Met Val Thr Phe Arg Asn Gln Ala Ser Arg Pro Tyr Ser
   1790 1795 1800
   Phe Tyr Ser Ser Leu Ile Ser Tyr Glu Glu Asp Gln Arg Gln Gly
   1805 1810 1815
   Ala Glu Pro Arg Lys Asn Phe Val Lys Pro Asn Glu Thr Lys Thr
   1820 1825 1830
   Tyr Phe Trp Lys Val Gln His His Met Ala Pro Thr Lys Asp Glu
   1835 1840 1845
   Phe Asp Cys Lys Ala Trp Ala Tyr Phe Ser Asp Val Asp Leu Glu
   1850 1855 1860
   Lys Asp Val His Ser Gly Leu Ile Gly Pro Leu Leu Val Cys His
   1865 1870 1875
   Thr Asn Thr Leu Asn Pro Ala His Gly Arg Gln Val Thr Val Gln
   1880 1885 1890
   Glu Phe Ala Leu Phe Phe Thr Ile Phe Asp Glu Thr Lys Ser Trp
   1895 1900 1905
   Tyr Phe Thr Glu Asn Met Glu Arg Asn Cys Arg Ala Pro Cys Asn
   1910 1915 1920
   Ile Gln Met Glu Asp Pro Thr Phe Lys Glu Asn Tyr Arg Phe His
   1925 1930 1935
   Ala Ile Asn Gly Tyr Ile Met Asp Thr Leu Pro Gly Leu Val Met
   1940 1945 1950
   Ala Gln Asp Gln Arg Ile Arg Trp Tyr Leu Leu Ser Met Gly Ser
   1955 1960 1965
   Asn Glu Asn Ile His Ser Ile His Phe Ser Gly His Val Phe Thr
   1970 1975 1980
   Val Arg Lys Lys Glu Glu Tyr Lys Met Ala Leu Tyr Asn Leu Tyr
   1985 1990 1995
   Pro Gly Val Phe Glu Thr Val Glu Met Leu Pro Ser Lys Ala Gly
   2000 2005 2010
   Ile Trp Arg Val Glu Cys Leu Ile Gly Glu His Leu His Ala Gly
   2015 2020 2025
   Met Ser Thr Leu Phe Leu Val Tyr Ser Asn Lys Cys Gln Thr Pro
   2030 2035 2040
   Leu Gly Met Ala Ser Gly His Ile Arg Asp Phe Gln Ile Thr Ala
   2045 2050 2055
   Ser Gly Gln Tyr Gly Gln Trp Ala Pro Lys Leu Ala Arg Leu His
   2060 2065 2070
   Tyr Ser Gly Ser Ile Asn Ala Trp Ser Thr Lys Glu Pro Phe Ser
   2075 2080 2085
   Trp Ile Lys Val Asp Leu Leu Ala Pro Met Ile Ile His Gly Ile
   2090 2095 2100
   Lys Thr Gln Gly Ala Arg Gln Lys Phe Ser Ser Leu Tyr Ile Ser
   2105 2110 2115
   Gln Phe Ile Ile Met Tyr Ser Leu Asp Gly Lys Lys Trp Gln Thr
   2120 2125 2130
   Tyr Arg Gly Asn Ser Thr Gly Thr Leu Met Val Phe Phe Gly Asn
   2135 2140 2145
   Val Asp Ser Ser Gly Ile Lys His Asn Ile Phe Asn Pro Pro Ile
   2150 2155 2160
   Ile Ala Arg Tyr Ile Arg Leu His Pro Thr His Tyr Ser Ile Arg
   2165 2170 2175
   Ser Thr Leu Arg Met Glu Leu Met Gly Cys Asp Leu Asn Ser Cys
   2180 2185 2190
   Ser Met Pro Leu Gly Met Glu Ser Lys Ala Ile Ser Asp Ala Gln
   2195 2200 2205
   Ile Thr Ala Ser Ser Tyr Phe Thr Asn Met Phe Ala Thr Trp Ser
       2210                    2215                    2220
   Pro Ser  Lys Ala Arg Leu His  Leu Gln Gly Arg Ser  Asn Ala Trp
       2225                    2230                    2235
   Arg Pro  Gln Val Asn Asn Pro  Lys Glu Trp Leu Gln  Val Asp Phe
       2240                    2245                    2250
   Gln Lys  Thr Met Lys Val Thr  Gly Val Thr Thr Gln  Gly Val Lys
       2255                    2260                    2265
   Ser Leu  Leu Thr Ser Met Tyr  Val Lys Glu Phe Leu  Ile Ser Ser
       2270                    2275                    2280
   Ser Gln  Asp Gly His Gln Trp  Thr Leu Phe Phe Gln  Asn Gly Lys
       2285                    2290                    2295
   Val Lys  Val Phe Gln Gly Asn  Gln Asp Ser Phe Thr  Pro Val Val
       2300                    2305                    2310
   Asn Ser  Leu Asp Pro Pro Leu  Leu Thr Arg Tyr Leu  Arg Ile His
       2315                    2320                    2325
   Pro Gln  Ser Trp Val His Gln  Ile Ala Leu Arg Met  Glu Val Leu
       2330                    2335                    2340
   Gly Cys  Glu Ala Gln Asp Leu  Tyr
       2345                    2350